(19) Europäisches Patentamt
European Patent Office
Office européen des brevets

(11) **EP 1 428 495 A1**

(12) **DEMANDE DE BREVET EUROPEEN**

(43) Date de publication:
**16.06.2004 Bulletin 2004/25**

(51) Int Cl.⁷: **A61K 7/043**

(21) Numéro de dépôt: **03292652.9**

(22) Date de dépôt: **23.10.2003**

(84) Etats contractants désignés:
**AT BE BG CH CY CZ DE DK EE ES FI FR GB GR HU IE IT LI LU MC NL PT RO SE SI SK TR**
Etats d'extension désignés:
**AL LT LV MK**

(30) Priorité: **13.12.2002 FR 0215857**

(71) Demandeur: **L'OREAL**
**75008 Paris (FR)**

(72) Inventeurs:
- **Mougin, Nathalie**
**75011 Paris (FR)**
- **Cazeneuve, Colette**
**75007 Paris (FR)**
- **Vicic, Marco**
**94360 Bry s/Marne (FR)**

(74) Mandataire: **Dodin, Catherine**
**L'OREAL - D.I.P.I.**
**25-29 Quai Aulagnier**
**92600 Asnières (FR)**

(54) **Composition de vernis à ongles comprenant un copolymère à gradient et procédé cosmétique de maquillage ou de soin des ongles**

(57) La présente demande concerne une composition de vernis à ongles comprenant un copolymère filmogène à gradient présentant un indice de polydispersité en masse (Ip) inférieur ou égal à 2,5, ladite composition étant apte à former un film présentant une vitesse de perte de masse inférieure à 1 mg/minute, lorsque le film est soumis à une abrasion effectuée avec l'abrasimètre TABER à 23 °C.

L'invention concerne également un procédé cosmétique de maquillage ou de soin des ongles comprenant l'application sur ceux-ci d'une composition cosmétique telle que définie ci-dessus.

EP 1 428 495 A1

## Description

La présente invention a trait à de nouvelles compositions cosmétiques notamment de maquillage, et plus particulièrement de vernis à ongles, comprenant des copolymères particuliers, notamment des copolymères amphiphiles à gradient, de préférence solubles ou dispersibles dans l'eau et/ou dans les solvants organiques, lesdites compositions présentant de bonnes propriétés de tenue.

Les compositions de vernis à ongles comprennent généralement, de façon connue, un polymère filmogène dans un milieu solvant organique ou un milieu aqueux. Le vernis forme après séchage un film coloré ou incolore sur les ongles et permet ainsi d'embellir ou de protéger les ongles contre les agressions extérieurs tels que les frottements, les rayures. Or, fréquemment, les vernis à ongles présentent une mauvaise tenue dans le temps : le film se détériore, notamment par écaillage ou décollement, au bout d'un ou deux jours. Une telle détérioration se produit souvent à l'extrémité de l'ongle. Lorsque le vernis est endommagé, l'utilisatrice doit alors retirer le vernis abîmé puis procéder à une nouvelle application de vernis. L'utilisatrice peut également retoucher le vernis endommagé en appliquant partiellement du vernis mais ce type de retouche ne conduit pas à un maquillage parfaitement esthétique. Si l'utilisatrice ne fait rien, le vernis endommagé nuit à l'aspect esthétique du maquillage et n'assure pas une bonne protection de l'ongle. D'autres vernis à ongles comme les vernis facilement pelable ou les vernis démaquillable à l'eau ne confèrent pas non plus une très bonne tenue dans le temps.

Par ailleurs, l'utilisation au sein d'une même composition, d'un mélange de plusieurs polymères de nature chimique très différente, chaque polymère apportant une des caractéristiques souhaitées, peut générer des problèmes de démixtion au sein de la composition, lesdites natures chimiques n'étant pas forcément compatibles.
L'utilisation de polymères statistiques, par exemple de polymères acryliques conventionnels obtenus par polymérisation radicalaire classique par mélange statistique de monomères, ne permet pas de résoudre ces problèmes de manière satisfaisante. En effet, les polymères statistiques connus dans l'art antérieur présentent une dispersité en composition des chaînes polymériques, ce qui conduit également à une démixtion des polymères au sein de la formule.

Un besoin existe donc de disposer de compositions de vernis à ongles permettant d'obtenir un film déposé sur les ongles présentant une tenue dans le temps satisfaisante, sans présenter de défaut inesthétique, lesdites compositions étant particulièrement stables et homogènes.
Le but de la présente invention est donc de proposer une telle composition de vernis à ongles présentant à la fois une bonne stabilité et de bonnes propriétés de tenue dans le temps, en particulier une bonne résistance aux frottements, à l'eau et/ou à l'écaillage.

Un objet de la présente invention est donc une composition de vernis à ongles comprenant, dans un milieu cosmétiquement acceptable, un copolymère filmogène à gradient comprenant au moins deux monomères différents, et présentant un indice de polydispersité en masse (Ip) inférieur ou égal à 2,5 de préférence compris entre 1,1 et 2,3, notamment entre 1,15 et 2,0, voire entre 1,2 et 1,9 ou 1,8, ladite composition étant apte à former un film présentant une vitesse de perte de masse inférieure à 1 mg/minute, de préférence inférieure à 0,8 mg/minute, et préférentiellement inférieure à 0,6 mg/minute, voire à 0,4 mg/minute, lorsque le film est soumis à une abrasion effectuée avec l'abrasimètre TABER à 23 °C.

Dans la présente demande, on entend par "polymère filmogène", un polymère apte à former à lui seul, ou en présence d'agent auxiliaire de filmification, un film continu et adhérent sur l'ongle, à une température allant de 20°C à 30°C.

Les copolymères selon l'invention sont des copolymères à gradient, qui comprennent au moins deux monomères différents, et qui présentent une polydispersité en masse faible et de préférence une polydispersité en composition faible.

Comme les copolymères selon l'invention présentent une faible dispersité en composition, toutes les chaînes présentant quasiment les mêmes structures, elles sont donc compatibles entre elles; il en résulte que les compositions cosmétiques comprenant ces copolymères ne présentent pas les inconvénients et limitations des compositions de l'art antérieur.
Notamment les copolymères selon l'invention présentent l'avantage d'être facilement manipulables dans l'eau ou dans un milieu solvant organique, tout en conservant des propriétés rhéologiques intéressantes.

La polydispersité en masse peut être illustrée à l'aide de l'indice de polydispersité en masse (Ip) du copolymère, qui est égal au rapport de la masse moléculaire moyenne en poids (Mw) sur la masse moléculaire moyenne en nombre (Mn).
Une faible dispersité en masse traduit des longueurs de chaînes approximativement identiques, ce qui est le cas pour les copolymères selon la présente invention.
Le copolymère à gradient selon l'invention possède un indice de polydispersité en masse inférieur ou égal à 2,5 de préférence compris entre 1,1 et 2,3, notamment entre 1,15 et 2,0, voire entre 1,2 et 1,9 ou 1,8.

Par ailleurs, la masse moléculaire moyenne en poids du copolymère à gradient est de préférence comprise entre 5000 g/mol et 1 000 000 g/mol, notamment entre 5500 g/mol et 800 000 g/mol, et encore mieux entre 6000 g/mol et 500 000 g/mol.
De préférence, la masse moléculaire moyenne en nombre du copolymère à gradient est comprise entre 5000 g/mol et 1 000 000 g/mol, notamment entre 5500 g/mol et 800 000 g/mol, et encore mieux entre 6000 g/mol et 500 000 g/mol.
On détermine les masses moléculaires moyennes en poids (Mw) et en nombre (Mn) par chromatographie liquide par perméation de gel (GPC), éluant THF, courbe d'étalonnage établie avec des étalons de polystyrène linéaire, détecteur réfractométrique).

Le copolymère à gradient selon l'invention présente également préférentiellement une faible dispersité en composition. Ceci signifie que toutes les chaînes de copolymères ont une composition (c'est-à-dire un enchaînement de monomères) approximativement analogue et sont donc homogènes en composition.
Afin de montrer que toutes les chaînes de copolymères ont une composition analogue, on utilisera avantageusement la chromatographie d'adsorption liquide (ou LAC) qui permet de séparer les chaînes de copolymères, non pas selon leur poids moléculaire mais selon leur polarité. Cette dernière reflète la composition chimique des polymères constituant le matériau, les monomères étant connus.
On pourra se reporter à la publication Macromolecules (2001), 34, 2667 qui décrit la technique LAC.
On peut notamment définir la polydispersité en composition à partir de la courbe de chromatographie d'adsorption (LAC) (courbe représentant la proportion de polymères en fonction du volume d'élution): si l'on nomme "$V^{1/2}$ min" la valeur minimum du volume d'élution à mi-hauteur de la courbe, et "$V^{1/2}$ max" la valeur maximum du volume d'élution à mi-hauteur de la courbe, la polydispersité en composition est considérée comme faible si la différence ($V^{1/2}$ max - $V^{1/2}$ min) est inférieure ou égale à 3,5, notamment comprise entre 1 et 2,8 et mieux entre 1,2 et 2,5.

Par ailleurs, la courbe LAC peut être définie par une courbe de Gauss de formule :

$$y = \frac{A}{W\sqrt{\frac{\pi}{2}}} \times e^{-2\frac{(x-x_0)^2}{w^2}} + y_o$$

dans laquelle :

- $x_0$ est la valeur de x (volume d'élution) au centre du pic
- w est égal à 2 fois la déviation standard de la distribution gaussienne (soit 2σ)

ou encore correspond à approximativement 0,849 fois la largeur du pic à mi-hauteur

- A représente la surface sous le pic
- $y_o$ est la valeur de y correspondant à $x_0$.

La dispersité en composition peut également être définie par la valeur de w telle que définie ci dessus. De préférence, ladite valeur w est comprise entre 1 et 3, notamment entre 1,1 et 2,3 et encore mieux entre 1,1 et 2,0.

Les copolymères à gradient selon l'invention peuvent être obtenus par polymérisation vivante ou pseudo-vivante.
Pour mémoire, nous rappelons que la polymérisation vivante est une polymérisation pour laquelle la croissance des chaînes polymériques ne cesse qu'avec la disparition du monomère. La masse moléculaire moyenne en nombre (Mn) croît avec la conversion. La polymérisation anionique est un exemple typique de polymérisation vivante. De telles polymérisations conduisent à des copolymères dont la dispersité en masse est faible c'est-à-dire à des polymères d'indice de polydispersité en masse (Ip) généralement inférieur à 2.
La polymérisation pseudo-vivante est, quant à elle, associée à la polymérisation radicalaire contrôlée. Parmi les principaux types de polymérisation radicalaire contrôlée, on peut citer :

- la polymérisation radicalaire contrôlée par des nitroxydes. On peut notamment se référer aux demandes de brevet WO96/24620 et WO00/71501 qui décrivent les outils de cette polymérisation et leur mise en oeuvre, ainsi qu'aux articles publiés par Fischer (Chemical Reviews, 2001, 101, 3581), par Tordo et Gnanou (J. Am. Chem. Soc. 2000, 122, 5929) et Hawker (J. Am. Chem. Soc. 1999, *121*, 3904);
- la polymérisation par transfert d'atome radicalaire, notamment décrite dans la demande WO96/30421 et qui procède par l'insertion réversible sur un complexe organométallique dans une liaison de type carbone-halogène;
- la polymérisation radicalaire contrôlée par des dérivés soufrés de type xanthate, dithioesters, trithiocarbonates ou carbamates, telle que décrite dans les demandes FR2821620, WO98/01478, WO99/35177, WO98/58974, WO99/31144, WO97/01478 et dans la publication de Rizzardo & al. (Macromolecules, 1998, 31, 5559).

La polymérisation radicalaire contrôlée désigne des polymérisations pour lesquelles les réactions secondaires qui conduisent habituellement à la disparition des espèces propageantes (réaction de terminaison ou transfert) sont rendues très peu probables par rapport à la réaction de propagation grâce à un agent de contrôle des radicaux libres. L'imperfection de ce mode de polymérisation réside dans le fait que lorsque les concentrations en radicaux libres deviennent importantes par rapport à la concentration en monomère, les réactions secondaires redeviennent déterminantes et tendent à élargir la distribution des masses.

Grâce à ces modes de polymérisation, les chaînes polymériques des copolymères à gradient selon l'invention croissent simultanément et donc incorporent à chaque instant les mêmes ratio de co-monomères. Toutes les chaînes possèdent donc les mêmes structures ou des structures proches, d'où une faible dispersité en composition. Ces chaînes possèdent également un indice de polydispersité en masse faible.
Les copolymères à gradient sont des copolymères présentant une évolution du ratio des différents monomères tout au long de la chaîne. La distribution dans les chaînes polymériques des co-monomères dépend de l'évolution pendant la synthèse des concentrations relatives des co-monomères.
Les copolymères selon l'invention comprennent au moins deux monomères différents dont la concentration le long de la chaîne polymérique change graduellement et de façon systématique et prédictible.
Ceci signifie que toutes les chaînes polymériques ont au moins un monomère Mi pour lequel, quelque soit la position normalisée x sur la chaîne polymérique, il y a une probabilité non nulle de retrouver ce monomère Mi le long de la chaîne.
Une des caractéristiques permettant de définir les copolymères à gradient est le fait qu'à tout instant de la polymérisation, toutes les chaînes sont soumises à la présence de l'ensemble de tous les monomères. Ainsi, dans le milieu réactionnel, la concentration de chaque monomère est toujours non nulle, à tout instant de la polymérisation.
Ceci permet de différencier les copolymères selon l'invention des polymères-blocs usuels dans lesquels l'évolution des monomères le long de la chaîne polymérique n'est pas systématique par exemple pour un dibloc AB, au sein de la séquence A, la concentration en l'autre monomère B est toujours nulle.
Dans le cas des polymères statistiques, l'évolution des monomères le long de la chaîne polymérique ne sera pas non plus graduelle, systématique et prédictible. Comme illustré par le schéma ci-après, un polymère statistique obtenu par polymérisation radicalaire classique de deux monomères se distingue d'un copolymère à gradient, par la répartition des monomères qui n'est pas identique sur toutes les chaînes, et par la longueur desdites chaînes qui n'est pas identique pour toutes les chaînes.

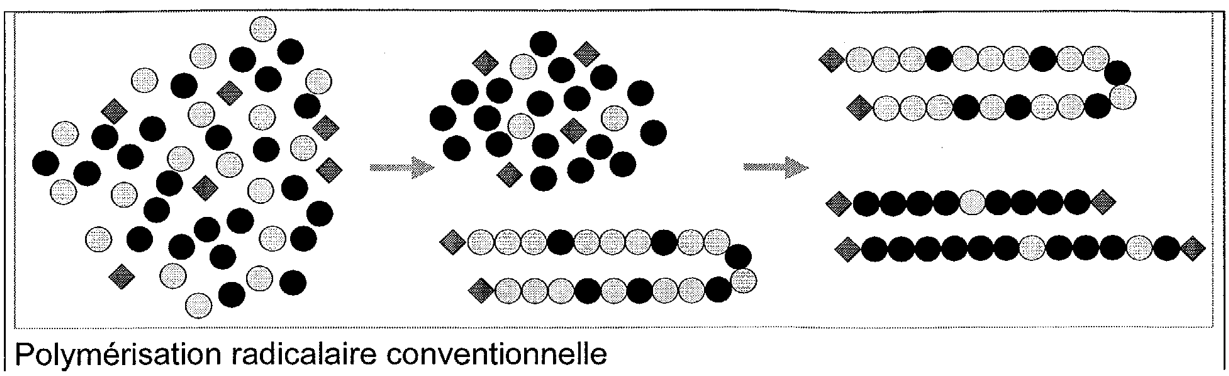

Polymérisation radicalaire conventionnelle

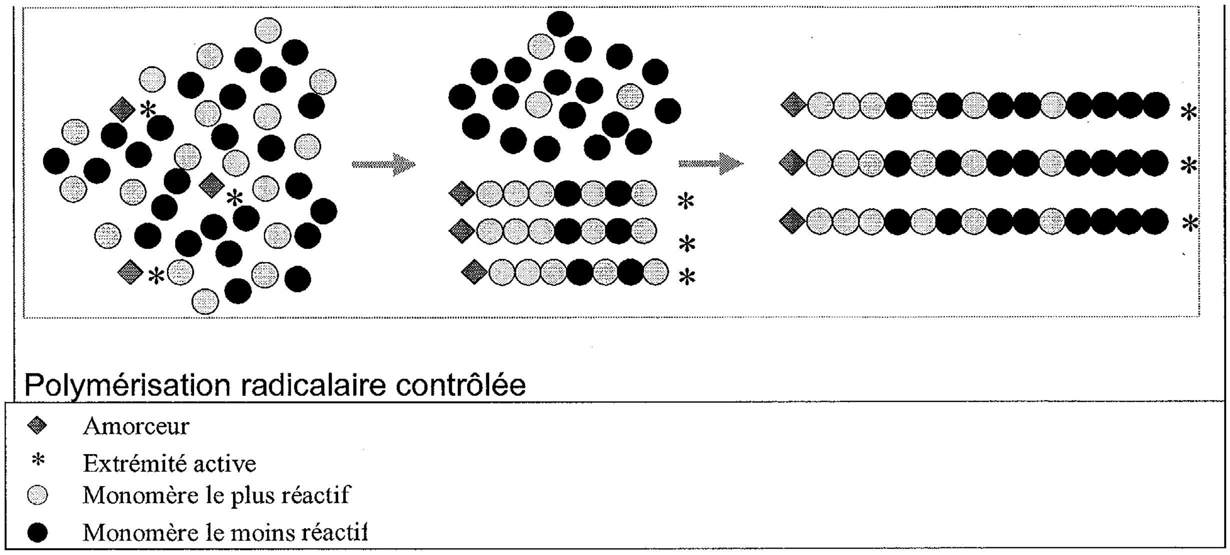


Pour une description théorique des copolymères à gradient, il est possible de se reporter aux publications suivantes :

T. Pakula et al., Macromol. Theory Simul. 5, 987-1006 (1996) ;
A. Aksimetiev et al. J. of Chem. Physics 111, n°5 ;
M. Janco, J. Polym. Sci., Part A: Polym. Chem. (2000), 38(15), 2767-2778 ;
M. Zaremski et al., Macromolecules (2000), 33(12), 4365-4372 ;
K. Matyjaszewski & al., J. Phys. Org. Chem. (2000), 13(12), 775-786 ;
Gray, Polym. Prepr. (Am. Chem. Soc., Div. Polym. Chem.) (2001), 42(2), 337-338 ;
K. Matyjaszewski, Chem. Rev. (Washington, D. C.) (2001), 101(9), 2921-2990.

Parmi les copolymères à gradient, on peut distinguer les copolymères à gradient naturel, et les copolymères à gradient artificiel.
Un copolymère à gradient naturel est un copolymère à gradient synthétisé en batch à partir d'un mélange initial des co-monomères. La distribution dans la chaîne des divers monomères suit une loi déduite de la réactivité relative et des concentrations initiales de monomères. Ces copolymères constituent la classe la plus simple de copolymères à gradient car c'est le mélange initial qui définit la propriété finale de produit.
Un copolymère à gradient artificiel est un copolymère dont on fait varier la concentration en monomères durant la synthèse par un artifice de procédé. Dans ce cas, on passe d'un mélange de monomères à un autre dans la chaîne du fait d'un changement subit et brusque des monomères dans le milieu réactionnel (stripping du premier mélange ou addition d'au moins un nouveau monomère). Il peut même y avoir une disparition nette d'un ou plusieurs des monomères au profit d'un ou plusieurs autres.
La caractérisation expérimentale du gradient est apportée par la mesure en cours de polymérisation de la composition chimique du polymère. Cette mesure est faite de façon indirecte en déterminant l'évolution de la teneur des différents monomères à tout instant. Elle peut être faite par RMN et UV, par exemple.
En effet, pour les polymères préparés par polymérisation vivante ou pseudo-vivante, la longueur des chaînes est linéairement liée à la conversion.
En prélevant un échantillon de la solution de polymérisation, à différents instants de la polymérisation et en mesurant la différence de teneur en chaque monomère, on accède ainsi à la composition du gradient.
Dans le polymère à gradient, la distribution des compositions des chaînes est étroite. En particulier il n'existe pas de recouvrement entre le pic du copolymère à gradient et ceux des homopolymères respectifs. Ceci signifie que le matériau obtenu en gradient est constitué de chaînes polymères de même composition alors qu'en polymérisation statistique classique, différentes sortes de chaîne coexistent, y compris celles des homopolymères respectifs.
Il est possible de caractériser les copolymères à gradient par un vecteur caractéristique de chaque copolymère.
En effet, sachant qu'il existe une infinité de polymères caractérisés par une composition chimique donnée, pour préciser un polymère il est possible de décrire la répartition des monomères le long de la chaîne. Ce qui implique une description

à plusieurs variables. Ce vecteur est un point de l'espace des compositions chimiques.
Le terme exact est que G est un vecteur dont les coordonnées sont les concentrations des monomères le long de la chaîne polymérique. Ces concentrations sont définies par les règles des coefficients de réactivité de chacun des monomères, et donc sont liées à la concentration des monomères libres pendant la synthèse : du moment que le monomère n'est pas en concentration nulle dans le mélange réactionnel, il n'est pas en concentration nulle dans le polymère.
Il est donc possible de caractériser les copolymères à gradient par la fonction G(x) qui définit le gradient de composition :

$$\vec{G}(x) = \Sigma\overrightarrow{[Mi](x)}$$

dans laquelle :

- x désigne une position normalisée sur la chaîne polymère et
- [Mi](x) est la concentration relative, en cette position x, du monomère Mi exprimée en % en moles.

La fonction G(x) décrit donc localement la composition du copolymère à gradient.
Deux copolymères peuvent avoir une composition globale équivalente mais des répartitions locales des monomères très différentes, donc des gradients différents.
Par exemple, dans le cas d'un dibloc AB (50/50), la fonction [A] vaut 1 jusqu'à x=1/2 puis 0 ensuite.
Les facteurs déterminant le gradient sont d'une part les coefficients de réactivité relatifs de chaque monomère (appelé $r_i$ pour le monomère Mi) qui dépendent principalement du type de procédé de synthèse employé (homogène, dispersé) et des solvants, et d'autre part les concentrations initiales de chacun des monomères, ainsi que les ajouts éventuels de monomères au cours de la polymérisation.
Ainsi, si l'on considère, à titre d'exemple, un copolymère à gradient de styrène (M1) ayant un coefficient de réactivité relatif $r_1$= 0,418 et de l'acide méthacrylique (M2), avec $r_2$=0,6, dans un système de polymérisation homogène :

- la variation des concentrations initiales en styrène et en acide méthacrylique permet l'obtention des différents copolymères à gradients ayant des chaînes de structures complètement différentes.
- lorsque la concentration initiale en acide méthacrylique est de 10% en poids, on obtient au final un copolymère à gradient très faible dont on ne peut attendre une nanostructuration.
- lorsque cette concentration initiale est de 20% en poids, on obtient un copolymère à gradient ayant une "tête" hydrophile et une "queue" hydrophobe avec un gradient suffisamment prononcé pour conduire à une nanostructuration.
- lorsque cette concentration est de 50% en poids, les monomères étant iso-réactifs dans ces conditions, le copolymère obtenu est du type alterné.
  Bien que les copolymères décrits soient tous des copolymères à gradient de styrène et d'acide méthacrylique, la différence de concentration initiale des monomères conduit à des chaînes de structures complètement différentes conférant aux copolymères des propriétés différentes. Cet exemple illustre donc l'importance des compositions initiales de monomère sur l'arrangement le long de la chaîne des différents monomères.

Dans le cas d'un copolymère à gradient styrène/acide méthacrylique, les différents polymères obtenus peuvent être schématisés ainsi, les boules blanches correspondant au styrène alors que les boules foncées correspondent à l'acide méthacrylique :

10% en acide méthacrylique initialement:

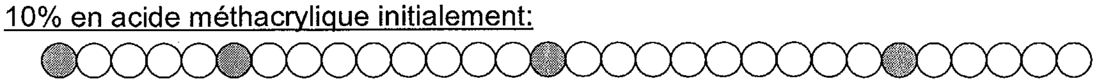

Copolymère à gradient très faible dont on ne peut attendre une nanostructuration.

20% en acide méthacrylique initialement:

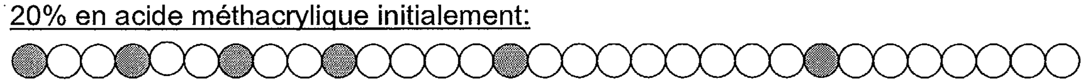

Copolymère à « tête » hydrophile et queue hydrophobe avec un gradient suffisamment prononcé pour conduire à une nanostructuration.

50% en acide méthacrylique initialement:

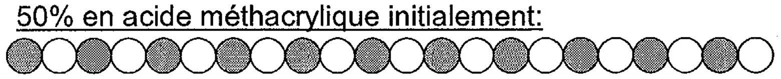

Les monomères étant isoréactifs dans ces conditions, le copolymère obtenu est du type alterné.

La structure de ces polymères peut être déterminée par la disparition de l'acide méthacrylique en fonction du taux de conversion.

Le copolymère à gradient selon l'invention comprend au moins deux monomères différents, qui peuvent être présents chacun à raison de 1 à 99% en poids par rapport au copolymère final, notamment à raison de 2-98% en poids, de préférence à raison de 5-95% en poids.

De préférence, le copolymère à gradient comprend au moins un monomère hydrophile, ou un mélange de monomères hydrophiles.
Ces monomères hydrophiles peuvent être présents à raison de 1 à 99% en poids, notamment 2-70% en poids, encore mieux 3-50% en poids, voire 4-30% en poids et encore mieux 5-25% en poids, par rapport au poids total du copolymère.

Dans la présente description, on désignera indifféremment par 'monomère hydrophile' les monomères dont les homopolymères sont solubles ou dispersibles dans l'eau, ou dont une forme ionique l'est.
Un homopolymère est dit hydrosoluble s'il forme une solution limpide lorsqu'il est en solution à 1 % en poids dans l'eau, à 25°C.
Un homopolymère est dit hydrodispersible si, à 1 % en poids dans l'eau, à 25°C, il forme une suspension stable de fines particules, généralement sphériques. La taille moyenne des particules constituant ladite dispersion est inférieure à 1 µm et, plus généralement, varie entre 5 et 400 nm, de préférence de 10 à 250 nm.
Ces tailles de particules sont mesurées par diffusion de lumière.

De préférence, le monomère hydrophile a une Tg supérieure ou égale à 20°C, notamment supérieure ou égale à 50°C, mais peut éventuellement avoir une Tg inférieur ou égale à 20°C.

Le copolymère à gradient peut également comprendre au moins un monomère hydrophobe, en particulier de monomère hydrophobe susceptible d'être rendu hydrophile après polymérisation, ou un mélange de tels monomères.
Le(s) monomère(s) hydrophobes peuvent être rendus hydrophile(s) par exemple par réaction chimique notamment par hydrolyse, ou par modification chimique, en particulier d'une fonction ester par incorporation de chaînes comportant un motif hydrophile, par exemple de type acide carboxylique.

Ces monomères hydrophobes peuvent être présents à raison de 1 à 99% en poids, notamment 30-98% en poids, encore mieux 50-97% en poids, voire 70-96% en poids, et encore mieux 75-95% en poids par rapport au poids total du copolymère.
De préférence, le monomère hydrophobe a une Tg supérieure ou égale à 20°C, notamment supérieure ou égale à 30°C, mais peut éventuellement avoir une Tg inférieur ou égale à 20°C.

De préférence, le copolymère à gradient selon l'invention comprend au moins un monomère ayant une Tg inférieure ou égale à 20°C, notamment comprise entre -150°C et 20°C, plus particulièrement comprise entre -130°C et 18°C, et encore mieux comprise entre -120°C et 15°C, ou un mélange de tels monomères.
Ces monomères de Tg $\leq$ 20°C peuvent être présents à raison de 1 à 99% en poids, notamment 20-90% en poids, encore mieux 30-85% en poids, voire 50-75% en poids, par rapport au poids total du copolymère.
Les monomères de Tg $\geq$ 20°C peuvent donc être présents à raison de 1 à 99% en poids, notamment 10-80% en poids, encore mieux 15-70% en poids, voire 25-50% en poids, par rapport au poids total du copolymère.
Dans la présente description, on désignera par 'monomère de Tg' les monomères dont l'homopolymère a une telle Tg, mesurée selon la méthode décrite ci-après.

Dans la présente invention, la Tg (ou température de transition vitreuse) est mesurée selon la norme ASTM D3418-97, par analyse enthalpique différentielle (DSC "Differential Scanning Calorimetry) sur calorimètre, sur une plage de température comprise entre -100°C et +150°C à une vitesse de chauffe de 10°C/min dans des creusets en aluminium de 150 µl.

Dans un mode de réalisation préféré, le copolymère à gradient selon l'invention comprend trois monomères différents, qui peuvent être présents à raison de 5-90% en poids chacun, notamment 7-86% en poids chacun par rapport au poids total du copolymère.
En particulier, le copolymère peut comprendre 5-25% en poids d'un premier monomère, 5-25% en poids d'un second monomère et 50-90% en poids d'un troisième monomère.
Préférentiellement, le copolymère selon l'invention peut comprendre 5-25% en poids d'un monomère hydrophile, 50-90% en poids d'un monomère de Tg inférieure ou égale à 20°C et 5-25% en poids d'un monomère additionnel.

Parmi les monomères hydrophiles susceptibles d'être employés dans la présente invention, on peut citer les

monomères suivants :

- les dérivés de (méth)acrylates d'aminoalkyles en C1-C6, et notamment les (méth)acrylates N,N-dialkyle en C1-C6 aminoalkyle en C1-C6 tels que le méthacrylate de N,N-diméthylaminoéthyle (MADAME) ou le méthacrylate de N, N-diéthylaminoéthyle (DEAMEA) ;
- les (méth)acrylamides N,N-dialkyle en C1-C4 éventuellement aminoalkyle en C1-C6, tels que la N,N-diméthylacrylamide, le N,N-diméthylaminopropylacrylamide (DMAPA) ou le N,N-diméthylaminopropylmethacrylamide (DMAPMA),
- les dialkyl diallylamines en C1-C8 tels que la diméthyldiallylamine;
- la vinylamine;
- les vinylpyridines et notamment la 2-vinylpyridine ou la 4-vinylpiridine ;

ainsi que leurs sels d'acides minéraux, ou d'acide organique, ou leurs formes quaternisées.
Parmi les acides minéraux, on peut citer l'acide sulfurique ou l'acide chlorhydrique, l'acide bromhydrique, iodhydrique, l'acide phosphorique, l'acide borique.
Parmi les acides organiques, on peut citer les acides comportant un ou plusieurs groupes carboxylique, sulfonique ou phosphonique. Il peut s'agir d'acides aliphatiques linéaires, ramifiés ou cycliques ou encore d'acides aromatiques. Ces acides peuvent comporter, en outre, un ou plusieurs hétéroatomes choisis parmi O et N, par exemple sous la forme de groupes hydroxyles.
Un exemple d'acide à groupe alkyle est l'acide acétique $CH_3COOH$ ou l'acide propionique. Un exemple de polyacide est l'acide téréphtalique. Des exemples d'hydroxyacides sont l'acide citrique et l'acide tartrique.
Les agents quaternisants peuvent être des halogénures d'alkyles tel que bromure de méthyle ou des sulfates d'alkyle tels le methylsulfate ou la propane sultone.

On peut encore citer :

- les acides carboxyliques, notamment mono- ou di-carboxyliques, éthyléniques tels que l'acide acrylique, l'acide méthacrylique, l'acide crotonique, l'acide itaconique, l'acide fumarique, l'acide maléique ;
- les anhydrides carboxyliques porteurs d'une liaison vinylique tels que l'anhydride maléique
- les acides sulfoniques éthyléniques tels que l'acide styrènesulfonique, l'acide acrylamidopropanesulfonique, et les sels de ceux-ci;
- l'acide vinylbenzoïque, l'acide vinylphosphonique et leurs sels.
- le sel de potassium de l'acryloyloxy-3-sulfopropyle, ou le composé de formule $CH_2=CHCOOCH_2OCH_2$ (OH) $CH_2SO_3^-Na^+$

Le neutralisant peut être une base minérale, telle que LiOH, NaOH, KOH, $Ca(OH)_2$, $NH_4OH$; ou une base organique, par exemple une amine primaire, secondaire ou tertiaire, notamment une alkylamine éventuellement hydroxylée comme la dibutylamine, la triéthylamine, la stearamine, ou bien l'amino-2-méthyl-2-propanol, la monoéthanolamine, la diéthanolamine, la stearamidopropyldimethylamine.

On peut encore citer :

- les amides des acides carboxyliques insaturés comme l'acrylamide ou le méthacrylamide et leur dérivés N-substitués tels que les (méth)acrylamides de N-alkyle en $C_1$-$C_4$ comme la N-méthylacrylamide; les (méth)acrylamides de N,N-dialkyle $C_1$-$C_4$, comme la N,N-diméthylacrylamide;
- les (méth)acrylates d'hydroxyalkyle notamment ceux dont le groupe alkyle a de 2 à 4 atomes de carbone, en particulier le (méth)acrylate d'hydroxyéthyle;
- les (méth)acrylates de polyéthylène glycol (5 à 100 OE) ou de glycol substitués ou non sur leur fonction terminale par des groupements alkyl, phosphates, phosphonates, sulfonate, par exemple l'acrylate de glycérol, le (méth) acrylate de methoxypolyethylene glycol (8 ou 12OE); le (méth)acrylate d'hydroxypolyethylene glycol ;
- les (méth)acrylates d'alkoxyalkyle tels que le (méth)acrylate d'éthoxyéthyle;
- les (méth)acrylates de polysaccharide comme l'acrylate de saccharose.
- les vinylamides telles que la vinyl acétamide; éventuellement cycliques en particulier les vinyllactames tels que la N-vinylpyrrolidone ou la N-vinylcaprolactame;
- les vinyléthers tels que le vinylmethyléther.

On peut encore citer :

- le methacrylamidopropoxy trimethylammoniumbetaine;
- le N,N-dimethyl-N-methacryloxyéthyl-N-(3-sulfopropyl)ammoniumbetaine,

- le 3-methacryloylethoxycarbonylpyridinium
- le composé de formule :

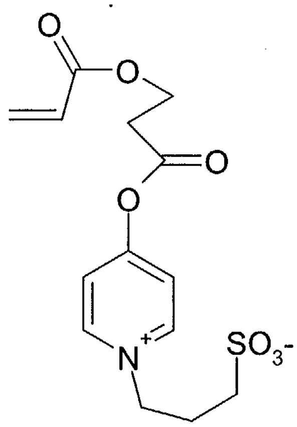

- le 4-vinylpyridyniumsulfopropylbetaine de formule :

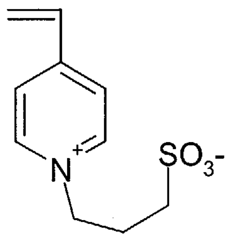

De préférence, les monomères hydrophiles sont choisis parmi le méthacrylate de N,N-diméthylaminoéthyle (MADAME), l'acide acrylique, l'acide méthacrylique, l'acide crotonique, l'acide styrènesulfonique, l'acide acrylamidopropanesulfonique, le diméthylaminopropylmethacrylamide (DMAPMA); le styrène sulfonate, l'acrylate d'hydroxyéthyle, l'acrylate de glycérol, le méthacrylate d'éthoxyéthyle, l'acrylate d'éthoxyéthyle, le (méth)acrylate de methoxypolyethylene glycol (8 ou 12OE); le (méth)acrylate d'hydroxypolyethylene glycol ; la N-vinylpyrrolidone, la N-vinylcaprolactame, l'acrylamide, la N,N-dimethylacrylamide.

Parmi les monomères hydrophobes susceptibles d'être rendus hydrophiles, notamment par hydrolyse, on peut citer les (méth)acrylates d'alkyles en C1-C4, tels que le (méth)acrylate de tertio-butyle ou le (méth)acrylate d'éthyle, qui conduisent à l'obtention de l'acide (méth)acrylique après hydrolyse.

Parmi les monomères dont l'homopolymère a une Tg inférieure ou égale à 20°C, et dont certains peuvent être hydrophiles, susceptibles d'être employés dans la présente invention, on peut citer :

- les hydrocarbures éthyléniques de 2 à 10 carbone, tels que l'éthylène, l'isoprène, et le butadiène ;
- les acrylates de formule $CH_2 = CHCOOR_1$, $R_1$ représentant un groupe hydrocarboné saturé ou non, de 1 à 12 carbones, linéaire ou ramifié à l'exception du groupe tertio-butyle, dans lequel se trouve(nt) éventuellement intercalé(s) un ou plusieurs hétéroatomes choisis parmi O, N, S, Si, ledit groupe alkyle pouvant en outre être éventuellement substitué par un ou plusieurs substituants choisis parmi les groupes hydroxyle et les atomes d'halogène (Cl, Br, I et F) ;
  des exemples de groupes $R_1$ sont les groupes méthyle, éthyle, propyle, butyle, isobutyle, hexyle, éthylhexyle, octyle, lauryle, isooctyle, isodécyle, hydroxyéthyle, hydroxypropyle, méthoxyéthyle, éthoxyéthyle, méthoxypropyle, éthylperfluorooctyle, et propylpolydiméthylsiloxane;
  $R_1$ peut aussi être un groupement $-(R'')x-(OC_2H_4)_n-OR'''$, avec x = 0 ou 1, R" = groupe hydrocarboné saturé ou non, de 1 à 12 carbones, linéaire ou ramifié, n = 5 à 100 et R''' = H ou $CH_3$; et notamment un groupement méthoxy (POE)8-stéaryle.
- les méthacrylates de formule : $CH_2=C(CH_3)-COOR_2$
  dans laquelle $R_2$ représente un groupe hydrocarboné saturé ou non, de 3 à 12 carbones, linéaire ou ramifié, dans lequel se trouve(nt) éventuellement intercalé(s) un ou plusieurs hétéroatomes choisis parmi O, N, S et Si, ledit groupe alkyle pouvant en outre être éventuellement substitué par un ou plusieurs substituants choisis parmi les

groupes hydroxyle et les atomes d'halogènes (Cl, Br, I, F) ;
des exemples de groupes $R_2$ sont hexyle, éthylhexyle, octyle, lauryle, isooctyle, isodécyle, dodécyle, méthoxyéthyle, méthoxypropyle, éthoxyéthyle; éthylperfluorooctyle, et propylpolydiméthylsiloxane;
$R_2$ peut aussi être un groupement $-(R'')x-(OC_2H_4)_n-OR'''$, avec x = 0 ou 1, R" = groupe hydrocarboné saturé ou non, de 1 à 12 carbones, linéaire ou ramifié, n = 5 à 100 et R''' = H ou $CH_3$; et notamment un groupement méthoxy (POE)8-stéaryle.

- les dérivés N ou N,N-substitués des amides d'acides carboxyliques insaturés en C1-12, notamment les (méth) acrylamides de N-alkyle en C1-12, tel que le N-octylacrylamide;
- les esters de vinyle de formule : $R_3$-CO-O-CH = $CH_2$ où $R_3$ représente un groupe alkyle de 2 à 12 carbone, linéaire ou ramifié, notamment le propionate de vinyle, le butyrate de vinyle, l'éthylhexanoate de vinyle, le néononanoate de vinyle, et le néododécanoate de vinyle ;
- les éthers de vinyle et d'alkyle ayant 1 à 12 carbones tels que l'éther de vinyle et de méthyle, et l'éther de vinyle et d'éthyle ;

De préférence, les monomères de Tg inférieure ou égale à 20°C sont choisis parmi :

- l'isoprène et le butadiène ;
- les acrylates de méthyle, d'éthyle, d'isobutyle, de n-butyle, d'éthylhexyle; de méthoxyéthyle, d'éthoxyéthyle; d'hydroxypolyéthylèneglycol;
- les méthacrylates d'éthoxyéthyle, d'hexyle, d'éthylhexyle; d'hydroxypolyéthylèneglycol;
- les (méth)acrylamides de N-alkyle en C6-12, tel que le N-octylacrylamide;
- les esters de vinyle de formule : $R_3$-CO-O-CH = $CH_2$ où $R_3$ représente un groupe alkyle de 6 à 12 carbones, linéaire ou ramifié, notamment le néononanoate de vinyle et le néododécanoate de vinyle.

Parmi les monomères dont l'homopolymère a une Tg supérieure ou égale à 20°C, et dont certains peuvent être hydrophiles, susceptibles d'être employés dans la présente invention, on peut citer :

- les composés vinyliques de formule : $CH_2$ = CH-$R_4$, où $R_4$ est un groupe hydroxyle ; un groupe -NH-C(O)-$CH_3$, un groupe -OC(O)-$CH_3$, un groupe cycloalkyle en $C_3$ à $C_8$ ; un groupe aryle en $C_6$ à $C_{20}$ ; un groupe aralkyle en $C_7$ à $C_{30}$ (groupe alkyle en $C_1$ à $C_4$) ; un groupe hétérocyclique de 4 à 12 chaînons contenant un ou plusieurs héréatomes choisis parmi O, N, et S ; un groupe hétérocyclylalkyle (alkyle en $C_1$ à $C_4$) tel qu'un groupe furfuryle ; lesdits groupes cycloalkyle, aryle, aralkyle, hétérocyclique, ou hétérocyclylalkyle pouvant être éventuellement substitué par un ou plusieurs substituants choisis parmi les groupes hydroxyles, les atomes d'halogène, et les groupes alkyles de 1 à 4 carbone, linéaires ou ramifiés dans lesquels se trouve(nt) éventuellement intercalé(s) un ou plusieurs hétéroatomes choisis parmi O, N, S et P, et lesdits groupes alkyle pouvant, en outre, être éventuellement substitués par un ou plusieurs substituants choisis parmi les groupes hydroxyles, et les atomes d'halogène (Cl, Br, I et F) ou Si. Des exemples de monomères vinyliques sont le vinylcyclohexane, le styrène et l'acétate de vinyle.
- les acrylates de formule $CH_2$ = CH-COO$R_5$, où $R_5$ est un groupe tertiobutyle, un groupe cycloalkyle en $C_3$ à $C_8$ ; un groupe aryle en $C_6$ à $C_{20}$ ; un groupe aralkyle en $C_7$ à $C_{30}$ (groupe alkyle en $C_1$ à $C_4$) ; un groupe hétérocyclique de 4 à 12 chaînons contenant un ou plusieurs hétéroatomes choisis parmi O, N, et S ; un groupe hétérocyclylalkyle (alkyl de $C_1$ à $C_4$), tel qu'un groupe furfuryle ; lesdits groupes cycloalkyle, aryle, aralkyle, hétérocyclique ou hétérocyclylalkyle pouvant être éventuellement substitué par un ou plusieurs substituants choisis parmi les groupes hydroxyles, les atomes d'halogène, et les groupes alkyles de 1 à 4 C linéaires ou ramifiés dans lesquels se trouve (nt) éventuellement intercalé(s) un ou plusieurs hétéroatomes choisis parmi O, N, S et P, lesdits groupes alkyle pouvant, en outre, être éventuellement substitués par un ou plusieurs substituants choisis parmi les groupes hydroxyle et les atomes d'halogène (Cl, Br, I et F) ou Si. Des exemples de monomères acrylate sont les acrylates de t-butylcyclohexyle, de tertiobutyle, de t-butylbenzyle, de furfuryle et d'isobornyle ;
- les méthacrylates de formule $CH_2$ = C($CH_3$)-COO$R_6$, où $R_6$ est un groupe alkyle de 1 à 4C, linéaire ou ramifié, tel qu'un groupe méthyle, éthyle, propyle ou isobutyle, ledit groupe alkyle pouvant en outre être éventuellement substitué par un ou plusieurs substituants choisis parmi les groupes hydroxyle et les atomes d'halogène (Cl, Br, I et F) ou Si; un groupe cycloalkyle en $C_3$ à $C_8$ ; un groupe aryle en $C_6$ à $C_{20}$ ; un groupe aralkyle en $C_7$ à $C_{30}$ (groupe alkyle en $C_1$ à $C_4$) ; un groupe hétérocyclique de 4 à 12 chaînons contenant un ou plusieurs hétéroatomes choisis parmi O, N, et S ; un groupe hétérocyclylalkyle (alkyle de 1 à 4 C), tel qu'un groupe furfuryle ; lesdits groupes cycloalkyle, aryle, aralkyle, ou hétérocyclique ou hétérocyclylalkyle pouvant être éventuellement substitué par un ou plusieurs substituants choisis parmi les groupes hydroxyles, les atomes d'halogène, et les groupes alkyles de 1 à 4 carbone, linéaires ou ramifiés, dans lequel se trouve(nt) éventuellement intercalé(s) un ou plusieurs hétéroatomes choisis parmi O, N, S et P, lesdits groupes alkyle pouvant, en outre, être éventuellement substitués par

un ou plusieurs substituants choisis parmi les groupes hydroxyle et les atomes d'halogène (Cl, Br, I et F).
Des exemples de monomères méthacrylate sont les méthacrylates de méthyle, d'éthyle, de n-butyle, d'isobutyle, de t-butylcyclohexyle, de t-butylbenzyle, de méthoxyéthyle de méthoxypropyle et d'isobornyle ;

- les (méth)acrylamides de formule : $CH_2=C(R')-CO-NR_7R_8$,

où $R_7$ et $R_8$ identiques ou différents représentent un atome d'hydrogène ou un groupe alkyle de 1 à 12 atomes de carbone linéaire ou ramifié, tel qu'un groupe n-butyle, t-butyle, isopropyle, isohexyle, isooctyle, ou isononyle, et R' désigne H ou méthyle.
Des exemples de monomères (méth)acrylamide sont le N-butylacrylamide, le N-t-butylacrylamide, le N-isopropylacrylamide, le N,N-diméthylacrylamide et le N,N-dibutylacrylamide.

Les monomères de Tg supérieure ou égale à 20°C préférés sont choisis parmi :

- les acrylates de furfuryle, d'isobornyle, de tertiobutyle, de tertiobutylecyclohexyle, de tertiobutylbenzyle;
- les méthacrylates de méthyle, de n-butyle, d'éthyle, d'isobutyle,
- le styrène, le styrène sulfonate;
- l'acétate de vinyle et le vinylcyclohexane.

L'homme du métier saura choisir les monomères et leurs quantités en fonction du résultat recherché, en se basant sur ses connaissances générales, notamment sur la réactivité relative de chaque monomère.
Ainsi, si l'on souhaite un copolymère ayant des motifs hydrophiles dans le coeur d'une chaîne polymère, on choisira préférentiellement un initiateur di-fonctionnel et un mélange de monomères tel que la réactivité des monomères hydrophiles est supérieure à celle des autres monomères.

Par ailleurs, on a constaté que les procédés de préparation employés permettaient d'ajuster et de moduler la ou les Tg du copolymère, et ainsi d'obtenir un copolymère à gradient ayant une ou plusieurs Tg donnée(s).

Les copolymères à gradient de l'invention peuvent être préparés par l'homme du métier suivant le mode opératoire suivant :

1/ On prépare un mélange des différents monomères, éventuellement dans un solvant, de préférence dans un réacteur sous agitation. On ajoute un initiateur de polymérisation radicalaire et un agent de contrôle de la polymérisation. Le mélange est mis de préférence sous atmosphère de gaz inerte par rapport à une polymérisation radicalaire tel que l'azote ou l'argon.
Comme solvant éventuel de polymérisation, on peut choisir les acétates d'alkyle tels que l'acétate de butyle ou l'acétate d'éthyle, des solvants aromatiques tels que le toluène, des solvants cétoniques tels que la méthyléthylcétone, des alcools tels que l'éthanol. Dans le cas où le mélange de monomères est miscible à l'eau, celle ci peut être avantageusement utilisée comme solvant ou co-solvant.
2/ On porte le mélange sous agitation à la température de polymérisation souhaitée. Cette température est de préférence choisie dans une gamme allant de 10°C à 160°C, de préférence de 25°C à 130°C.
Le choix de la température de polymérisation est de préférence optimisé en fonction de la composition chimique du mélange de monomères. Ainsi, les monomères ayant des constantes cinétiques de propagation très élevée et une affinité pour l'agent de contrôle plus faible seront de préférence polymérisés à basse température (par exemple dans le cas d'une proportion importante de dérivés méthacryliques, on préfèrera une polymérisation à température comprise entre 25° et 80°C).
3/ Le milieu de polymérisation est éventuellement modifié pendant la polymérisation, avant d'atteindre 90% de conversion des monomère initiaux, par ajout supplémentaire d'un ou plusieurs monomères, notamment du mélange initial. Cet ajout peut être réalisé de différentes manières, pouvant aller de l'addition brutale en une seule fois à l'addition continue sur la durée totale de la polymérisation.
4/ La polymérisation est arrêtée lorsque le taux de conversion souhaité est atteint. De cette conversion dépend la composition globale du copolymère. De préférence, on arrête la polymérisation après avoir atteint au moins 50% de conversion, préférentiellement après avoir atteint au moins 90% de conversion.
5/ Les éventuels monomères résiduels peuvent être éliminés par toute méthode connue, telle que par évaporation, ou par ajout d'une quantité d'initiateur classique de polymérisation tel que les dérivés peroxydiques ou azoïques.

Dans un premier mode de réalisation, l'agent de contrôle de la polymérisation susceptible d'être employé est un nitroxyde de formule (I), seul ou en mélange :

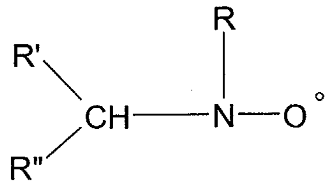

(I)

dans laquelle :

- R et R', indépendamment l'un de l'autre, sont des groupements hydrocarbonés saturés (alkyles) linéaires ou ramifiés, comportant 1 à 40 atomes de carbone, éventuellement substitués par un ou plusieurs groupements choisis parmi $-OR_3$, $-COOR_3$ et $-NHR_3$ (avec $R_3$ représentant H ou un groupement hydrocarboné saturé (alkyle) linéaire ou ramifié, comportant 1 à 40 atomes de carbone), R et R' pouvant en outre être reliés de manière à former un cycle; En particulier R et R' sont des groupements alkyle linéaires ou ramifiés comportant 1 à 12 atomes de carbone, notamment des groupements méthyle, éthyle, propyle, n-butyle, iso-butyle, ter-butyle, pentyle. De préférence, R et R' sont tous les deux des groupements ter-butyle.
- R" est un groupement monovalent de masse molaire (Mw) supérieure à 16 g/mol, notamment un groupement phosphoré de formule :

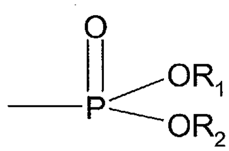

dans laquelle $R_1$ et $R_2$, indépendamment l'un de l'autre, sont des groupements hydrocarbonés saturés (alkyle) linéaires ou ramifiés, comportant 1 à 40 atomes de carbone, éventuellement substitués par un ou plusieurs groupements choisis parmi $-OR_3$, $-COOR_3$ et $-NHR_3$ (avec $R_3$ représentant H ou un groupement hydrocarboné saturé (alkyle) linéaire ou ramifié, comportant 1 à 40 atomes de carbone), $R_1$ et $R_2$ pouvant en outre être reliés de manière à former un cycle. En particulier $R_1$ et $R_2$ sont des groupements alkyle linéaires ou ramifiés comportant 1 à 12 atomes de carbone, notamment des groupements méthyle, éthyle, propyle, n-butyle, iso-butyle, ter-butyle, pentyle. De préférence, R1 et R2 sont tous les deux des groupements éthyle.

L'initiateur de polymérisation radicalaire peut être choisi parmi tous les initiateurs de polymérisation usuels, tels que les composés de type azoïque et notamment l'azobisisobutyronitrile, ou de type peroxyde tels que les peroxydes organiques ayant 6-30 atomes de carbone, notamment le peroxyde de benzoyle.

De préférence, on respecte un ratio molaire nitroxyde/initiateur compris entre 1 et 2,5; ce ratio peut être compris entre 2 et 2,5 lorsque l'on considère qu'une mole d'initiateur donne naissance à deux moles de chaînes polymères, et peut être compris entre 1 et 1,25 pour des initiateurs monofonctionnels.

Dans un second mode de réalisation particulier, on peut employer comme initiateur de polymérisation radicalaire des alcoxyamines de formule (II) qui peuvent être avantageusement choisies pour initier la polymérisation et libérer en même temps le nitroxyde contrôlant cette polymérisation.

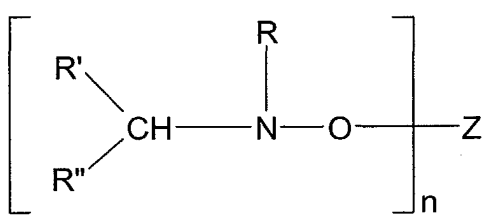

(II)

dans laquelle :

- R, R' et R" ont les significations données ci-dessus pour le nitroxyde de formule (I),
- n est un entier inférieur ou égal à 8, de préférence compris entre 1 et 3;
- Z est un radical monovalent ou multivalent, notamment un radical styryle, acryle ou méthacryle.

On peut également ajouter à l'alcoxyamine de formule (II), un nitroxyde de formule (I), dans une proportion allant de 0 à 20% molaires par rapport aux moles de fonctions alcoxyamines (une mole d'alcoxyamine multivalente apporte un nombre de fonctions alcoxyamine proportionnel à sa valence), de manière à améliorer la qualité du contrôle de polymérisation.

L'homme du métier saura choisir l'initiateur en fonction des besoins de l'application. Ainsi, un initiateur monofonctionnel conduira à des chaînes dissymétriques, alors que 'un initiateur poly-fonctionnel conduira à des macromolécules ayant une symétrie à partir d'un coeur.

Les copolymères à gradient selon l'invention peuvent être présents dans les compositions cosmétiques de vernis en une quantité de 0,1 à 60% en poids, de préférence 0,2 à 40% en poids, notamment 1 à 35% en poids, voire 5-30% en poids, par rapport au poids total de la composition.

Les copolymères peuvent être présents dans la composition sous forme solubilisée, par exemple dans l'eau ou un solvant organique, ou bien sous forme de dispersion aqueuse ou organique.

Un polymère est dit soluble lorsqu'il forme, à 1% en poids, une solution limpide à 25°C.

Il est dit dispersible lorsqu'il forme, à 1% en poids, à 25°C, une suspension stable de fines particules, généralement sphériques et dont la taille moyenne est de préférence inférieure à 1 micron, par exemple comprise entre 5 et 400 nm, de préférence 10 à 250 nm, mesurée par diffusion de la lumière.

Il est possible de préparer une solution ou dispersion aqueuse ou organique du copolymère directement par mélange du polymère avec l'eau ou le solvant organique, éventuellement en chauffant.

Lorsque le polymère comporte des motifs hydrophiles, notamment, on peut préparer une solution ou dispersion aqueuse en dissolvant ledit polymère dans un solvant organique de température d'ébullition inférieur à l'eau (par exemple l'acétone ou la méthyléthylcétone), à un taux de solide compris entre 20 et 90% en poids.

Lorsque les monomères hydrophiles sont de type acide, on peut ajouter à la solution organique, une solution de préférence au moins 1 M de base, telle qu'un sel d'ion hydroxonium ($OH^-$), une amine (l'ammoniac), un sel de carbonate ($CO_3^{2-}$) ou d'hydrogénocarbonate ($HCO_3^-$) ou de neutralisant organique. Dans le cas de monomères hydrophiles de type amine, on peut ajouter une solution, de préférence au moins 1M, d'acide. On ajoute alors l'eau à la solution sous vive agitation dans une proportion telle que le taux de solide obtenu soit compris entre 1 et 80% en poids. Eventuellement, l'eau peut être remplacée par un mélange hydroalcoolique, dans des proportions allant de 99/1 à 50/50. On évapore le solvant en agitant la solution à 100°C. La concentration se poursuit jusqu'à l'obtention du taux de solide désiré.

La vitesse de perte de masse du film de vernis à ongles est mesurée selon le protocole décrit ci-après.

On dépose une couche de la composition sur un support métallique (acier) pour obtenir après séchage pendant 24 heures à 23±2°C et à 55±5% d'humidité relative, un film ayant une épaisseur d'environ 100µm (après séchage). Le film est soumis à une abrasion pendant 60 minutes à l'aide de l'abrasimètre TABER (référence 5130 Abraser) muni de deux roues abrasives vendue sous la dénomination CS10F par la société Taber Industries et en appliquant sur chaque roue une force de 2,5 N. Pendant l'opération d'abrasion, on mesure la vitesse de la perte de masse de film sur la période de 60 minutes.

Le film de vernis à ongles selon l'invention présente une vitesse de perte de masse inférieure à 1 mg/minute, de préférence inférieure à 0,8 mg/minute, et préférentiellement inférieure à 0,6 mg/minute, voire à 0,4 mg/minute.

Avantageusement, le film de vernis à ongles présente une perte de brillance, après 10 secondes d'abrasion avec l'abrasimètre TABER décrit précédemment, inférieure ou égale à 14%, et de préférence inférieure ou égale à 12%, voire inférieure ou égale à 8%. La perte de brillance correspond au rapport (Bo-B)/Bo férieure ou égale à 8%. La perte de brillance correspond au rapport (Bo-B)/Bo en %, dans lequel B représente la brillance du film après abrasion et Bo représente la brillance du film avant abrasion La brillance du film est mesurée à l'aide d'un brillancemètre BYK-GARDNER à un angle de faisceau lumineux de 60°, 1 heure après l'abrasion.

Selon un mode de réalisation de l'invention, la composition de vernis à ongles selon l'invention est apte à former un film ayant un module d'Young allant de 10 à 200 MPa, de préférence allant de 10 à 100 MPa, et plus préférentiellement allant de 10 à 50 MPa.

On mesure les propriétés mécaniques du film de vernis à ongles en traction monotone selon la norme ASTM Standards, volume 06.01 D 2370-92 'Standard Test Method for Tensile Properties of Organic Coatings' . On découpe une éprouvette dans un film libre ayant une épaisseur de 150 ± 50 µm obtenu après 48 heures de séchage à 23 ± 2°C et 55 ± 5% d'humidité relative d'une couche de vernis à ongles déposée sur une matrice en téflon. L'éprouvette est de forme haltère, de longueur utile 33 mm et de largeur utile 6 mm. La section (s) de l'éprouvette est alors définie comme : s=largeur x épaisseur ($mm^2$), cette section (s) sera utilisée pour le calcul de la contrainte .

Les essais sont réalisés sur un appareil de traction équipé d'un extensomètre optique pour la mesure du déplacement

et commercialisé sous l'appellation Zwick Z010. Les mesures sont réalisées dans les mêmes conditions de température et d'humidité que pour le séchage, c'est-à-dire à une température de 23 ± 2°C et une humidité relative de 55 ± 5%. Les éprouvettes sont étirées à une vitesse de déplacement de 500 mm/min.
On impose donc une vitesse de déplacement et on mesure simultanément la longueur (L) de l'éprouvette et la force (f) nécessaire pour imposer cette longueur.
La longueur (L) est mesurée avec un extensomètre optique à l'aide de pastilles adhésives placées sur l'éprouvette haltère. La distance initiale entre ces 2 pastilles définie la longueur utile Lo utilisée pour calculer la déformation ε = (L/Lo)x100 exprimée en %.
Il est ainsi obtenu une courbe contrainte σ.(= f/s) en fonction de la déformation ε, l'essai étant conduit jusqu'à rupture de l'éprouvette.
Le module d'Young (module d'élasticité), exprimé en MPa, correspond à la pente de la courbe σ = f(ε), considérée dans la partie linéaire de la courbe (début de l'essai).

Avantageusement, le polymère filmogène est insoluble dans l'eau à 25°C, c'est-à-dire qu'il est soluble à moins de 1% en poids dans l'eau à 25°C (solubilité inférieure à 1% en poids). Le polymère est de préférence soluble dans les solvants organiques, comme l'acétate d'éthyle, l'acétate de butyle ou l'acétate de méthyle (solubilité supérieure à 90% en poids à 25 °C).

Les compositions cosmétiques selon l'invention comprennent, outre lesdits copolymères, un milieu cosmétiquement acceptable, c'est-à-dire un milieu compatible avec les ongles.

La composition peut ainsi comprendre en outre un polymère filmogène additionnel notamment choisi parmi les polymères synthétiques de type radicalaire
ou de type polycondensat, les polymères d'origine naturelle et leurs mélanges, en particulier les polymères acryliques, les polyuréthanes, les polyesters, les polyamides, les polyurées, les polymères cellulosiques comme la nitrocellulose. On peut encore citer les résines comme les résines sulfonamides, les résines alkyde, les esters de cellulose tel que l'acétobutyrate de cellulose, l'acétate de cellulose, l'acétopropionate de cellulose.
Ce polymère filmogène additionnel, seul ou en mélange, peut être présent en une teneur allant de 0,01 à 50% en poids, par rapport au poids total de la composition, et de préférence allant de 1 à 30% en poids.

La composition de vernis à ongles selon l'invention peut également comprendre un agent auxiliaire de filmification pour améliorer les propriétés filmogènes du vernis.
L'agent auxiliaire de filmification peut être choisi parmi tous les composés connus de l'homme du métier comme étant susceptibles de remplir la fonction recherchée, et peut notamment être choisi parmi les agents plastifiants.
Comme exemple d'agent plastifiant, on peut citer :

- les citrates tels que le citrate de triéthyle, le citrate de tributyle, l'acétylcitrate de triéthyle, l'acétylcitrate de tributyle, l'acétylcitrate de triéthyl-2 hexyle;
- les phtalates tels que le phtalate de diéthyle, le phtalate de dibutyle, le phtalate de dioctyle, le phtalate de dipentyle, le phtalate de diméthoxyéthyle;
- le phosphate de tricrésyle, le benzoate de benzyle, le phosphate de tributyle, l'acétylricinoléate de butyle, l'acétylricinoléate de glycéryle, le glycolate de butyle, le phosphate de tributoxyéthyle, le phosphate de triphényle, le tartrate de dibutyle, le camphre, le triacétate de glycérol, le N-éthyl-o,p-toluènesulfonamide,
- et leurs mélanges.

L'agent plastifiant peut être présent dans la composition en une teneur allant de 0,01 à 10% en poids, par rapport au poids total de la composition, et de préférence allant de 0,1 à 5% en poids. Avantageusement, le plastifiant est présent dans la composition selon un rapport pondéral polymère(s) filmogène(s) / plastifiant allant de 1,5 à 3.

La composition selon l'invention peut comprendre un milieu aqueux, un milieu hydroorganique ou un milieu solvant organique, de préférence un milieu solvant organique. Elle peut être notamment anhydre.
Le milieu aqueux de la composition peut être constitué essentiellement d'eau. La teneur en eau dans la composition peut aller de 10 % à 95 % en poids, par rapport au poids total de la composition, de préférence de 40 % à 90 % en poids, et mieux de 60% à 85% en poids.
Lorsque la composition comprend un milieu aqueux, le polymère filmogène peut y être présent sous forme de particules solides dispersées dans le milieu aqueux ou sous forme solubilisée.
La composition peut comprendre également un solvant organique, notamment miscible à l'eau, comme les monoalcools ayant 1 à 5 atomes de carbone, les glycols ayant 2 à 8 atomes de carbone, les cétones en C3-C4, les aldéhydes en C2-C4, notamment en une teneur pouvant aller de 0,1 % à 15 % en poids, par rapport au poids total de la composition.
Le milieu solvant organique ou hydroorganique de la composition peut comprendre un ou plusieurs solvants organiques choisis parmi :

- les cétones liquides à température ambiante tels que méthyléthylcétone, méthylisobutylcétone, diisobutylcétone,

l'isophorone, la cyclohexanone, l'acétone;

- les alcools liquides à température ambiante tels que l'éthanol, l'isopropanol, le n-propanol, le n-butanol, le diacétone alcool, le 2-butoxyéthanol, le cyclohexanol;
- les glycols liquides à température ambiante tels que l'éthylène glycol, le propylène glycol, le pentylène glycol, le glycérol ;
- les éthers de propylène glycol liquides à température ambiante tels que le monométhyléther de propylène glycol, l'acétate de monométhyl éther de propylène glycol, le mono n-butyl éther de dipropylène glycol ;
- les esters à chaîne courte (ayant de 3 à 8 atomes de carbone au total) tels que l'acétate d'éthyle, l'acétate de méthyle, l'acétate de propyle, l'acétate de n-butyle, l'acétate d'isopentyle ;
- les éthers liquides à température ambiante tels que le diéthyléther, le diméthyléther ou le dichlorodiéthyléther ;
- les alcanes liquides à température ambiante tels que le décane, l'heptane, le dodécane, l'isododécane, le cyclohexane ;
- les composés cycliques aromatiques liquides à température ambiante tels que le toluène et le xylène ;
- les aldéhydes liquides à température ambiante tels que le benzaldéhyde, l'acétaldéhyde,
- et leurs mélanges.

La teneur en solvant organique dans la composition peut aller de 10 % à 95 % en poids, par rapport au poids total de la composition, de préférence de 40 % à 90 % en poids, et mieux de 60 % à 85 % en poids.

La composition peut comprendre un agent épaississant, notamment pour conférer à la composition une consistance permettant une bonne application de la composition sur les ongles.

L'agent épaississant est plus particulièrement un épaississant de solvant organique et peut être choisi parmi les silices hydrophobes, telles que celles décrites dans le document EP-A-898960, et par exemple commercialisées sous les références "AEROSIL R812®" par la société Degussa, "CAB-O-SIL TS-530®", "CAB-O-SIL TS-610®", "CAB-O-SIL TS-720®"par la société Cabot, "AEROSIL R972®", "AEROSIL R974®" par la société Degussa ; les argiles telles que la montmorillonite, l'hectorite stéaralkonium, la bentonite stéaralkonium ; les alkyléther de polysaccharides (notamment dont le groupe alkyle comporte 1 à 24 atomes de carbone, de préférence 1 à 10, mieux 1 à 6, et plus spécialement 1 à 3) tels que ceux décrits dans le document EP-A-898958, et par exemple commercialisés sous les dénominations "N-HANCE-AG 200®" et "N-HANCE AG 50®" par la société Aqualon.

L'agent épaississant peut être présent dans la composition selon l'invention en une teneur allant de 0,05 % à 10 % en poids, par rapport au poids total de la composition, et de préférence allant de 0,1 % à 3 % en poids.

La composition selon l'invention peut en outre comprendre une ou plusieurs matières colorantes choisies parmi les colorants hydrosolubles, les colorants liposolubles, et les matières colorantes pulvérulentes comme les pigments, les nacres et les paillettes bien connues de l'homme du métier.

Les matières colorantes peuvent être présentes, dans la composition, en une teneur allant de 0,01 à 50% en poids, par rapport au poids de la composition, de préférence de 0,05 à 30% en poids, et mieux de 0,1 à 25% en poids.

Par pigments, il faut comprendre des particules de toute forme, blanches ou colorées, minérales ou organiques, insolubles dans le milieu physiologique, destinées à colorer la composition. Par nacres, il faut comprendre des particules de toute forme irisées, notamment produites par certains mollusques dans leur coquille ou bien synthétisées.

Les pigments peuvent être blancs ou colorés, minéraux et/ou organiques. On peut citer, parmi les pigments minéraux, le dioxyde de titane, éventuellement traité en surface, les oxydes de zirconium ou de cérium, ainsi que les oxydes de zinc, de fer (noir, jaune ou rouge) ou de chrome, le violet de manganèse, le bleu outremer, l'hydrate de chrome et le bleu ferrique, les poudres métalliques comme la poudre d'aluminium, la poudre de cuivre.

Parmi les pigments organiques, on peut citer le noir de carbone, les pigments de type D & C, et les laques à base de carmin de cochenille, de baryum, strontium, calcium, aluminium.

Les pigments nacrés peuvent être choisis parmi les pigments nacrés blancs tels que le mica recouvert de titane, ou d'oxychlorure de bismuth, les pigments nacrés colorés tels que le mica titane recouvert avec des oxydes de fer, le mica titane recouvert avec notamment du bleu ferrique ou de l'oxyde de chrome, le mica titane recouvert avec un pigment organique du type précité ainsi que les pigments nacrés à base d'oxychlorure de bismuth.

Parmi les colorants hydrosolubles, on peut citer le sel disodique de ponceau, le sel disodique du vert d'alizarine, le sel trisodique d'amarante, le sel disodique de tartrazine, le sel monosodique de rhodamine, le sel disodique de fuchsine, la xanthophylle, le bleu de méthylène.

Les colorants liposolubles peuvent être par exemple le rouge Soudan, le DC Red 17, le DC Green 6, le β-carotène, l'huile de soja, le brun Soudan, le DC Yellow 11, le DC Violet 2, le DC orange 5, le jaune de quinoléine.

Les colorants peuvent être présents dans la composition en une teneur pouvant aller de 0,01 % à 6 % en poids, par rapport au poids total de la composition, de préférence allant de 0,05 % à 3 % en poids.

Les pigments, les nacres et les paillettes peuvent être présents dans la composition, notamment la composition de base et/ou de surface, en une teneur allant de 0,01 % à 25 % en poids, par rapport au poids de la composition, de préférence de 0,05 % à 15 % en poids

La composition selon l'invention peut également comprendre des ingrédients couramment utilisés en cosmétique, tels que les charges, les agents d'étalement, les agents mouillants, les agents dispersants, les anti-mousses, les conservateurs, les filtres UV, les actifs, les tensioactifs, les agents hydratants, les parfums, les stabilisants, les antioxydants, les vitamines, les oligo-éléments, les agents alcalinisants ou acidifiants, les céramides, ou leurs mélanges. Bien entendu, l'homme du métier veillera à choisir ce ou ces éventuels composés complémentaires, et/ou leur quantité, de manière telle que les propriétés avantageuses de la composition selon l'invention ne soient pas, ou substantiellement pas, altérées par l'adjonction envisagée.

La composition de vernis à ongles peut être employée comme base pour vernis, comme produit de maquillage des ongles, comme composition de finition, encore appelée "top-coat" en terminologie anglosaxonne, à appliquer sur le produit de maquillage des ongles ou bien encore comme produit de soin cosmétique des ongles. Ces compositions peuvent s'appliquer sur les ongles d'êtres humains ou bien encore sur des faux ongles.

L'invention a aussi pour objet un procédé cosmétique de maquillage ou de soin des ongles comprenant l'application sur ceux-ci d'une composition cosmétique telle que définie précédemment.

L'invention est illustrée plus en détail dans les exemples suivants.

Dans ces exemples, on utilise comme agent de contrôle de la polymérisation le nitroxyde stable, nommé SG1, de formule :

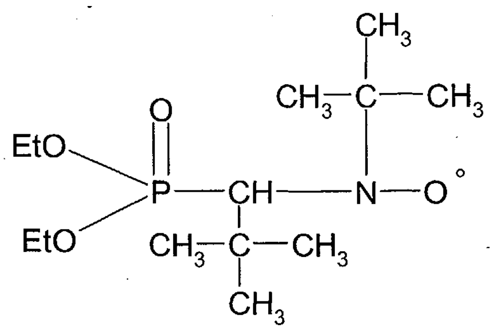

Les initiateurs de polymérisation mentionnés dans les exemples sont des alcoxyamines nommés 'DIAMS' et 'MONAMS' qui correspondent aux formules suivantes :

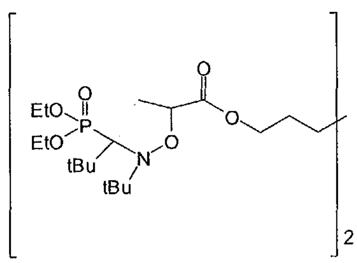

DIAMS

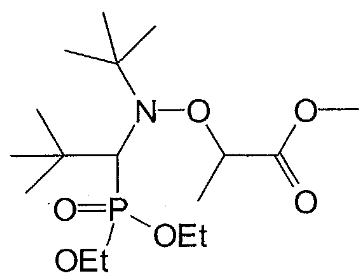

MONAMS

**Exemple 1 : synthèse en masse de copolymère à gradient**

Le mélange de réactifs est le suivant :

- MONAMS : 3,0 g
- SG1 : 0,18 g
- Acrylate d'éthyle : 480 g soit 80% en poids / poids total de monomères
- Styrène : 60 g soit 10% en poids / poids total de monomères
- Acide méthacrylique : 60 g soit 10% en poids / poids total de monomères

L'ensemble des constituants est mélangé, en l'absence de solvant, sous atmosphère d'azote, puis chauffé à une température maintenue entre 110 et 115°C pendant 198 minutes. La réaction est arrêtée à un taux de conversion

de 60%.

Le calcul du gradient par simulation donne la courbe ci-dessous. La prédiction théorique donne 30% d'incorporation du mélange (styrène/acide méthacrylique) et 70% d'acrylate d'éthyle.

La validité de ce modèle est donné par le suivi des concentrations relatives des trois monomères par chromatographie gazeuse et analyse RMN des polymères.
Par ces méthodes, on constate qu'à 60% de conversion, la composition chimique finale du copolymère est la suivante (% en poids): 68,4% d'acrylate d'éthyle, 16,1% de styrène et 15,5% d'acide méthacrylique selon la RMN sur la courbe calculée (69%).

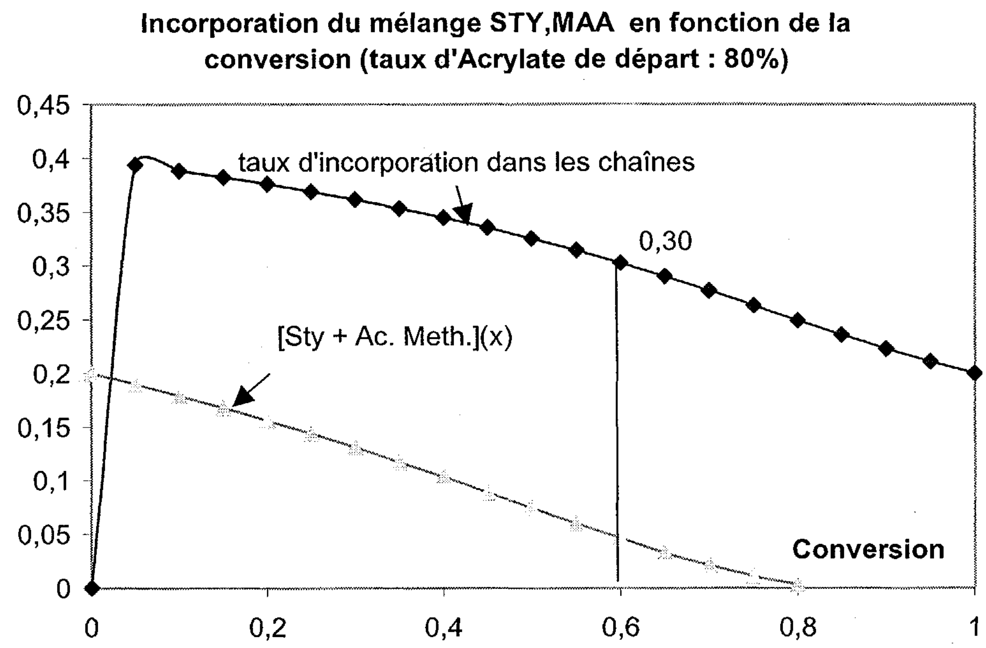


Par LAC, la trace du polymère montre la faible polydispersité de la composition chimique des chaînes.

La mesure des masses par chromatographie d'exclusion stérique conduit aux résultats suivants :
Mn= 32140 g/mol et Mw=51700 g/mol, d'où un indice de polydispersité Ip =1,6.
La dispersité en composition (ou w) est de 1,6.

Une représentation schématique du copolymère obtenu peut être la suivante :

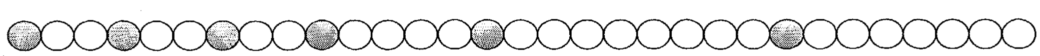

dans laquelle les sphères foncées désignent les enchaînements styrène/acide méthacrylique, et les sphères blanches désignent les enchaînements acrylate d'éthyle.

**<u>Exemple 2 : synthèse en masse de copolymère à gradient</u>**

Selon le mode opératoire décrit à l'exemple 1, on a préparé différents copolymères à partir du mélange de réactifs suivant :

- MONAMS : 3,0 g
- SG1 : 0,18 g
- Styrène : 60 g
- Acide méthacrylique : 60 g
- Acrylate (ou mélange d'acrylate) : 480 g

| Exemple | Acrylate | Caractéristiques du co-copolymère | Composition finale du copolymère (% en poids) |
|---|---|---|---|
| 2a | Acrylate de butyle | Mn= 31100 g/mol<br>Mw= 52930 g/mol<br>Ip = 1,7 | Styrène :18<br>Ac. méthacrylique : 22<br>Acrylate de butyle : 60 |
| 2b | Acrylate de méthyle | Mn= 32750 g/mol<br>Mw= 61470 g/mol<br>Ip = 1,88 | Styrène :20<br>Ac. méthacrylique : 21<br>Acrylate de méthyle: 59 |
| 2c | Mélange 50/50 en poids acrylate de butyle/acrylate d'éthyle | Mn= 29690 g/mol<br>Mw= 51630 g/mol<br>Ip = 1,74 | Styrène :18<br>Ac. méthacrylique : 16<br>Acrylates : 66 |

**Exemple 3 : synthèse en présence de solvant**

La même synthèse qu'à l'exemple 1 est réalisée, mais en présence de solvant.
Le mélange de réactifs est le suivant :

- MONAMS : 3,43 g
- SG1 : 0,2 g
- Acrylate d'éthyle : 336 g
- Styrène : 42 g
- Acide méthacrylique : 42 g
- Toluène : 180 g

L'ensemble des constituants est mélangé, dans le toluène comme solvant, sous atmosphère d'azote, puis chauffé à une température maintenue entre 110 et 115°C, pendant 198 minutes.
Le taux de conversion final est de 82%, et le taux de solide obtenu est de 57,2% en poids.
On détermine les résultats analytiques ci-après :
Mn = 30570 g/mol, Mw= 50500 g/mol et Ip = 1,65. La dispersité en composition (ou w) est de 2,0.
La composition finale du copolymère est donnée par chromatographie d'adsorption liquide (LAC), qui indique la similitude de composition avec le copolymère préparé à l'exemple 1 et l'absence d'homopolymère dans les matériaux.

**Exemple 4 : synthèse en présence de solvant**

On réalise, selon le procédé de l'exemple 3, à 120°C et durant 400 minutes, la synthèse d'un nouveau copolymère mais dans un solvant différent : la méthyléthylcétone.
La composition initiale du mélange est :

- MONAMS : 4,893 g
- SG1 : 0,2881 g
- Acrylate d'éthyle : 293,8 g
- Acrylate de méthyle : 32, 66 g
- Styrène : 76,8 g
- Acide méthacrylique : 76,8 g
- Méthyléthylcétone : 120 g

Le taux de conversion finale est de 99%, le taux de solide obtenu est de 79,9%. On détermine les résultats analytiques ci-après :

Mn = 30500 g/mol
Mw= 58000 g/mol
Ip = 1,9

L'incorporation des monomères au cours du temps est mesurée en suivant par chromatographie gazeuse les taux de monomères résiduels (en %) au cours du temps (en minute):

| temps | | 0 | 75 | 130 | 190 | 290 | 400 |
|---|---|---|---|---|---|---|---|
| Conversion Globale | | 0 | 16 | 30,5 | 49,5 | 85,4 | 99 |
| residuels (%) | AMe | 5,45 | 5,1 | 3,75 | 3,75 | 1,6 | 0,13 |
| | AE | 48,95 | | | | 17,95 | 1,2 |
| | AMA | 12,8 | 12,15 | 4,6 | 2 | 0,35 | 0,08 |
| | S | 12,8 | 12,46 | 6,7 | 3,92 | 0,15 | 0,007 |

- Acrylate d'éthyle : AE
- Acrylate de méthyle : AMe
- Styrène : S
- Acide méthacrylique AMA

Le taux de résiduel total est calculé en tenant compte du solvant quantifié par le taux de solide.

On note que chaque monomère est présent tout au long de la réaction. Le gradient déterminé pour chaque monomère peut alors être calculé et donne les courbes suivantes :

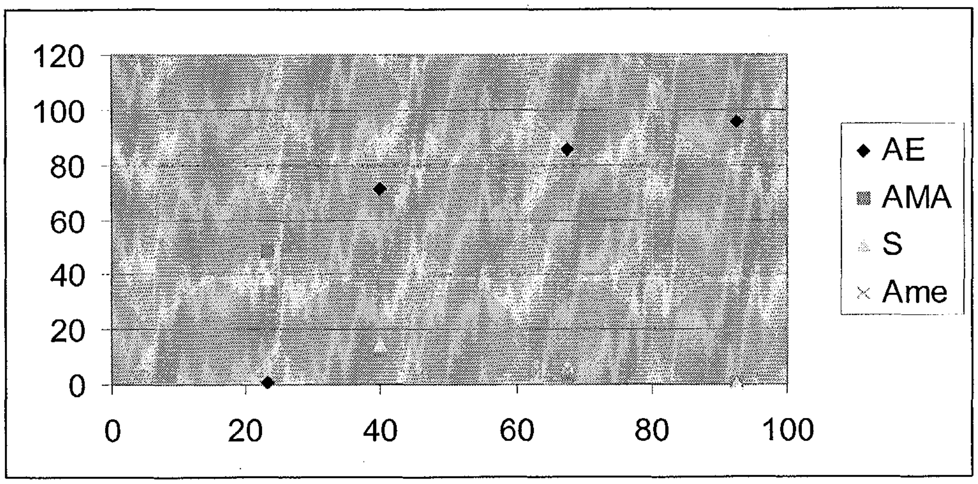


La composition finale du copolymère est la suivante :

- acrylate d'éthyle : 34% en poids
- acrylate de méthyle : 34% en poids
- styrène : 16% en poids
- acide méthacrylique : 16% en poids

**Exemple 5**

On solubilise les copolymères des exemples 1, 2a et 2c dans l'acétate de butyle, de manière à obtenir une solution ayant un taux de matière sèche de 10% en poids.
La solution obtenue est ensuite appliquée sur les ongles. On obtient après séchage un film de vernis qui a les caractéristiques suivantes, mesurées selon les protocoles décrits précédemment dans la description :

| | Vitesse de perte de masse (mg/min) | Perte de brillance (%) |
|---|---|---|
| Exemple 1 | 0,3 | 6 ± 2 |

(suite)

| | Vitesse de perte de masse (mg/min) | Perte de brillance (%) |
|---|---|---|
| Exemple 2a | 0,3 | 6 ± 1 |
| Exemple 2c | 0,4 | 7 ± 1 |

**Exemple 6**

On prépare une composition de vernis à ongles ayant la composition suivante :

- polymère de l'exemple 1 23,8 g en matière active
- Acétate de Butyle 25,0 g
- Isopropanol 10,7 g
- Hexylène Glycol 2,5 g
- pigment (DC RED 7 Lake) 1 g
- Hectorite modifiée (Bentone® 27V d'Elementis) 1,3 g

Après application sur les ongles, ce vernis a été jugé comme présentant de très bonnes propriétés de tenue et de résistance aux chocs.

**Revendications**

1. Composition de vernis à ongles comprenant, dans un milieu cosmétiquement acceptable, un copolymère filmogène à gradient comprenant au moins deux monomères différents, et présentant un indice de polydispersité en masse (Ip) inférieur ou égal à 2,5 de préférence compris entre 1,1 et 2,3, notamment entre 1,15 et 2,0, voire entre 1,2 et 1,9 ou 1,8, ladite composition étant apte à former un film présentant une vitesse de perte de masse inférieure à 1 mg/minute, de préférence inférieure à 0,8 mg/minute, et préférentiellement inférieure à 0,6 mg/minute, voire à 0,4 mg/minute, lorsque le film est soumis à une abrasion effectuée avec l'abrasimètre TABER à 23 °C.

2. Composition selon la revendication 1, dans laquelle la masse moléculaire moyenne en poids du copolymère à gradient est de préférence comprise entre 5000 g/mol et 1 000 000 g/mol, notamment entre 5500 g/mol et 800 000 g/mol, et encore mieux entre 6000 g/mol et 500 000 g/mol.

3. Composition selon l'une des revendications précédentes, dans laquelle la masse moléculaire moyenne en nombre du copolymère à gradient est comprise entre 5000 g/mol et 1 000 000 g/mol, notamment entre 5500 g/mol et 800 000 g/mol, et encore mieux entre 6000 g/mol et 500 000 g/mol.

4. Composition selon l'une des revendications précédentes, dans laquelle le copolymère est tel que toutes les chaînes polymériques ont au moins un monomère Mi pour lequel, quelque soit la position normalisée x sur la chaîne polymérique, il y a une probabilité non nulle de retrouver ce monomère Mi le long de la chaîne.

5. Composition selon l'une des revendications précédentes, dans laquelle le copolymère est tel que sur la courbe de chromatographie d'adsorption (LAC), représentant la proportion de polymères en fonction du volume d'élution, la différence ($V^{1/2}$ max - $V^{1/2}$ min) est inférieure ou égale à 3,5, notamment comprise entre 1 et 2,8 et mieux entre 1,2 et 2,5, avec "$V^{1/2}$ min" étant la valeur minimum du volume d'élution à mi-hauteur de la courbe, et "$V^{1/2}$ max" étant la valeur maximum du volume d'élution à mi-hauteur de la courbe.

6. Composition selon l'une des revendications précédentes, dans laquelle le copolymère à gradient comprend au moins deux monomères différents, qui sont présents chacun à raison de 1 à 99% en poids par rapport au copolymère final, notamment à raison de 2-98% en poids, de préférence à raison de 5-95% en poids.

7. Composition selon l'une des revendications précédentes, dans laquelle le copolymère à gradient comprend au moins un monomère hydrophile, ou un mélange de monomères hydrophiles, qui peut être présent à raison de 1 à 99% en poids, notamment 2-70% en poids, encore mieux 3-50% en poids, voire 4-30% en poids, et encore mieux 5-25% en poids, par rapport au poids total du copolymère.

**8.** Composition selon l'une des revendications précédentes, dans laquelle le copolymère à gradient comprend au moins un monomère dont l'homopolymère a une Tg inférieure ou égale à 20°C, notamment comprise entre -150°C et 20°C, plus particulièrement comprise entre -130°C et 18°C, et encore mieux comprise entre -120°C et 15°C, ou un mélange de tels monomères.

**9.** Composition selon la revendication 8, dans laquelle le monomère dont l'homopolymère a une Tg ≤ 20°C est présent à raison de 1 à 99% en poids, notamment 20-90% en poids, encore mieux 30-85% en poids, voire 50-75% en poids, par rapport au poids total du copolymère.

**10.** Composition selon l'une des revendications précédentes, dans laquelle le copolymère à gradient comprend au moins un monomère hydrophile choisi parmi :

- les dérivés de (méth)acrylates d'aminoalkyles en C1-C4, et notamment les (méth)acrylates N,N-dialkyle en C1-C4 aminoalkyle en C1-C6 tels que le méthacrylate de N,N-diméthylaminoéthyle (MADAME) ou le méthacrylate de N,N-diéthylaminoéthyle (DEAMEA) ;
- les (méth)acrylamides N,N-dialkyle en C1-C4 éventuellement aminoalkyle en C1-C6, tels que la N,N-diméthylacrylamide, le N,N-diméthylaminopropylacrylamide (DMAPA) ou le N,N-diméthylaminopropylmethacrylamide (DMAPMA),
- les dialkyl dallylamines en C1-C8 tels que la diméthyldiallylamine;
- la vinylamine;
- les vinylpyridines et notamment la 2-vinylpyridine ou la 4-vinylpiridine ;

ainsi que leurs sels d'acides minéraux, ou d'acide organique, ou leurs formes quaternisées.

- les acides carboxyliques, notamment mono- ou di-carboxyliques, éthyléniques tels que l'acide acrylique, l'acide méthacrylique, l'acide crotonique, l'acide itaconique, l'acide fumarique, l'acide maléique ;
- les anhydrides carboxyliques porteurs d'une liaison vinylique tels que l'anhydride maléique
- les acides sulfoniques éthyléniques tels que l'acide styrènesulfonique, l'acide acrylamidopropanesulfonique, et leurs sels;
- l'acide vinylbenzoïque, l'acide vinylphosphonique et leurs sels,
- le sel de potassium de l'acryloyloxy-3-sulfopropyle, ou le composé de formule $CH_2$=$CHCOOCH_2OCH_2$ (OH) $CH_2SO_3^-Na^+$
- les amides des acides carboxyliques insaturés comme l'acrylamide ou le méthacrylamide et leur dérivés N-substitués tels que les (méth)acrylamides de N-alkyle en $C_1$-$C_4$ comme la N-méthylacrylamide; les (méth) acrylamides de N,N-dialkyle $C_1$-$C_4$, comme la N,N-diméthylacrylamide;
- les (méth)acrylates d'hydroxyalkyle notamment ceux dont le groupe alkyle a de 2 à 4 atomes de carbone, en particulier le (méth)acrylate d'hydroxyéthyle;
- les (méth)acrylates de polyéthylène glycol (5 à 100 OE) ou de glycol substitués ou non sur leur fonction terminale par des groupements alkyl, phosphates, phosphonates, sulfonate, par exemple l'acrylate de glycérol, le (méth)acrylate de methoxypolyethylene glycol (8 ou 12OE); le (méth)acrylate d'hydroxypolyethylene glycol ;
- les (méth)acrylates d'alkoxyalkyle tels que le (méth)acrylate d'éthoxyéthyle;
- les (méth)acrylates de polysaccharide comme l'acrylate de saccharose.
- les vinylamides telles que la vinyl acétamide; éventuellement cycliques en particulier les vinyllactames tels que la N-vinylpyrrolidone ou la N-vinylcaprolactame;
- les vinyléthers tels que le vinylmethyléther.
- le methacrylamidopropoxy trimethylammoniumbetaine;
- le N,N-dimethyl-N-methacryloxyéthyl-N-(3-sulfopropyl)ammoniumbetaine,
- le 3-methacryloylethoxycarbonylpyridinium
- le composé de formule :

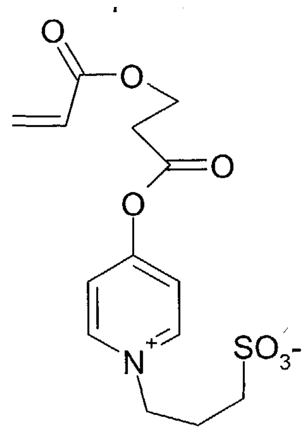

- le 4-vinylpyridyniumsulfopropylbetaine de formule :

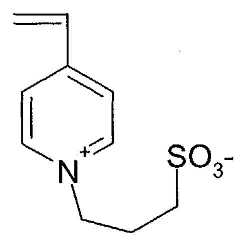

**11.** Composition selon l'une des revendications précédentes, dans laquelle le copolymère à gradient comprend au moins un monomère hydrophile choisi parmi le méthacrylate de N,N-diméthylaminoéthyle (MADAME), l'acide acrylique, l'acide méthacrylique, l'acide crotonique, l'acide styrènesulfonique, l'acide acrylamidopropanesulfonique, le diméthylaminopropylmethacrylamide (DMAPMA); le styrène sulfonate, l'acrylate d'hydroxyéthyle, l'acrylate de glycérol, le méthacrylate d'éthoxyéthyle, l'acrylate d'éthoxyéthyle, le (méth)acrylate de methoxypolyethylene glycol (8 ou 12OE); le (méth)acrylate d'hydroxypolyethylene glycol ; la N-vinylpyrrolidone, la N-vinylcaprolactame, l'acrylamide, la N,N-dimethylacrylamide.

**12.** Composition selon l'une des revendications précédentes, dans laquelle le copolymère à gradient comprend au moins un monomère choisi parmi les (méth)acrylates d'alkyles en C1-C4, tels que le (méth)acrylate de tertio-butyle ou le (méth)acrylate d'éthyle, qui conduisent à l'obtention de l'acide (méth)acrylique après hydrolyse.

**13.** Composition selon l'une des revendications précédentes, dans laquelle le copolymère à gradient comprend au moins un monomère dont l'homopolymère a une Tg inférieure ou égale à 20°C, choisi parmi :

- les hydrocarbures éthyléniques de 2 à 10 carbone, tels que l'éthylène, l'isoprène, et le butadiène ;
- les acrylates de formule $CH_2 = CHCOOR_1$, $R_1$ représentant un groupe hydrocarboné saturé ou non, de 1 à 12 carbones, linéaire ou ramifié à l'exception du groupe tertio-butyle, dans lequel se trouve(nt) éventuellement intercalé(s) un ou plusieurs hétéroatomes choisis parmi O, N, S, Si, ledit groupe alkyle pouvant en outre être éventuellement substitué par un ou plusieurs substituants choisis parmi les groupes hydroxyle et les atomes d'halogène (Cl, Br, I et F) ;
  $R_1$ peut aussi être un groupement $-(R'')x-(OC_2H_4)_n-OR'''$, avec x = 0 ou 1, R" = groupe hydrocarboné saturé ou non, de 1 à 12 carbones, linéaire ou ramifié, n = 5 à 100 et R''' = H ou $CH_3$;
- les méthacrylates de formule : $CH_2=C(CH_3)-COOR_2$ , dans laquelle $R_2$ représente un groupe hydrocarboné saturé ou non, de 3 à 12 carbones, linéaire ou ramifié, dans lequel se trouve(nt) éventuellement intercalé(s) un ou plusieurs hétéroatomes choisis parmi O, N, S et Si, ledit groupe alkyle pouvant en outre être éventuellement substitué par un ou plusieurs substituants choisis parmi les groupes hydroxyle et les atomes d'halogènes (Cl, Br, I, F) ; $R_2$ peut aussi être un groupement $-(R'')x-(OC_2H_4)_n-OR'''$, avec x = 0 ou 1, R" = groupe hydrocarboné saturé ou non, de 1 à 12 carbones, linéaire ou ramifié, n = 5 à 100 et R''' = H ou $CH_3$;
- les dérivés N ou N,N-substitués des amides d'acides carboxyliques insaturés en C1-12, notamment les (méth) acrylamides de N-alkyle en C1-12, tel que le N-octylacrylamide;

- les esters de vinyle de formule : $R_3$-CO-O-CH = $CH_2$ où $R_3$ représente un groupe alkyle de 2 à 12 carbone, linéaire ou ramifié, notamment le propionate de vinyle, le butyrate de vinyle, l'éthylhexanoate de vinyle, le néononanoate de vinyle, et le néododécanoate de vinyle ;
- les éthers de vinyle et d'alkyle ayant 1 à 12 carbones tels que l'éther de vinyle et de méthyle, et l'éther de vinyle et d'éthyle ;

**14.** Composition selon l'une des revendications précédentes, dans laquelle le copolymère à gradient comprend au moins un monomère dont l'homopolymère a une Tg inférieure ou égale à 20°C, choisi parmi :

- l'isoprène et le butadiène ;
- les acrylates de méthyle, d'éthyle, d'isobutyle, de n-butyle, d'éthylhexyle; de méthoxyéthyle, d'éthoxyéthyle; d'hydroxypolyéthylèneglycol;
- les méthacrylates d'éthoxyéthyle, d'hexyle, d'éthylhexyle; d'hydroxypolyéthylèneglycol;
- les (méth)acrylamides de N-alkyle en C6-12, tel que le N-octylacrylamide;
- les esters de vinyle de formule : $R_3$-CO-O-CH = $CH_2$ où $R_3$ représente un groupe alkyle de 6 à 12 carbones, linéaire ou ramifié, notamment le néononanoate de vinyle et le néododécanoate de vinyle.

**15.** Composition selon l'une des revendications précédentes, dans laquelle le copolymère à gradient comprend au moins un monomère dont l'homopolymère a une Tg supérieure ou égale à 20°C, choisi parmi :

- les composés vinyliques de formule : $CH_2$ = CH-$R_4$, où $R_4$ est un groupe hydroxyle ; un groupe -NH-C(O)-$CH_3$, un groupe -OC(O)-$CH_3$, un groupe cycloalkyle en $C_3$ à $C_8$ ; un groupe aryle en $C_6$ à $C_{20}$ ; un groupe aralkyle en $C_7$ à $C_{30}$ (groupe alkyle en $C_1$ à $C_4$) ; un groupe hétérocyclique de 4 à 12 chaînons contenant un ou plusieurs héréatomes choisis parmi O, N, et S ; un groupe hétérocyclylalkyle (alkyle en $C_1$ à $C_4$) tel qu'un groupe furfuryle ; lesdits groupes cycloalkyle, aryle, aralkyle, hétérocyclique, ou hétérocyclylalkyle pouvant être éventuellement substitué par un ou plusieurs substituants choisis parmi les groupes hydroxyles, les atomes d'halogène, et les groupes alkyles de 1 à 4 carbone, linéaires ou ramifiés dans lesquels se trouve(nt) éventuellement intercalé(s) un ou plusieurs hétéroatomes choisis parmi O, N, S et P, et lesdits groupes alkyle pouvant, en outre, être éventuellement substitués par un ou plusieurs substituants choisis parmi les groupes hydroxyles, et les atomes d'halogène (Cl, Br, I et F) ou Si.
- les acrylates de formule $CH_2$ = CH-$COOR_5$, où $R_5$ est un groupe tertiobutyle, un groupe cycloalkyle en $C_3$ à $C_8$ ; un groupe aryle en $C_6$ à $C_{20}$ ; un groupe aralkyle en $C_7$ à $C_{30}$ (groupe alkyle en $C_1$ à $C_4$) ; un groupe hétérocyclique de 4 à 12 chaînons contenant un ou plusieurs hétéroatomes choisis parmi O, N, et S ; un groupe hétérocyclylalkyle (alkyl de $C_1$ à $C_4$), tel qu'un groupe furfuryle ; lesdits groupes cycloalkyle, aryle, aralkyle, hétérocyclique ou hétérocyclylalkyle pouvant être éventuellement substitué par un ou plusieurs substituants choisis parmi les groupes hydroxyles, les atomes d'halogène, et les groupes alkyles de 1 à 4 C linéaires ou ramifiés dans lesquels se trouve(nt) éventuellement intercalé(s) un ou plusieurs hétéroatomes choisis parmi O, N, S et P, lesdits groupes alkyle pouvant, en outre, être éventuellement substitués par un ou plusieurs substituants choisis parmi les groupes hydroxyle et les atomes d'halogène (Cl, Br, I et F) ou Si.
- les méthacrylates de formule $CH_2$ = C($CH_3$)-$COOR_6$, où $R_6$ est un groupe alkyle de 1 à 4C, linéaire ou ramifié, tel qu'un groupe méthyle, éthyle, propyle ou isobutyle, ledit groupe alkyle pouvant en outre être éventuellement substitué par un ou plusieurs substituants choisis parmi les groupes hydroxyle et les atomes d'halogène (Cl, Br, I et F) ou Si; un groupe cycloalkyle en $C_3$ à $C_8$ ; un groupe aryle en $C_6$ à $C_{20}$ ; un groupe aralkyle en $C_7$ à $C_{30}$ (groupe alkyle en $C_1$ à $C_4$) ; un groupe hétérocyclique de 4 à 12 chaînons contenant un ou plusieurs hétéroatomes choisis parmi O, N, et S ; un groupe hétérocyclylalkyle (alkyle de 1 à 4 C), tel qu'un groupe furfuryle ; lesdits groupes cycloalkyle, aryle, aralkyle, ou hétérocyclique ou hétérocyclylalkyle pouvant être éventuellement substitué par un ou plusieurs substituants choisis parmi les groupes hydroxyles, les atomes d'halogène, et les groupes alkyles de 1 à 4 carbone, linéaires ou ramifiés, dans lequel se trouve(nt) éventuellement intercalé(s) un ou plusieurs hétéroatomes choisis parmi O, N, S et P, lesdits groupes alkyle pouvant, en outre, être éventuellement substitués par un ou plusieurs substituants choisis parmi les groupes hydroxyle et les atomes d'halogène (Cl, Br, I et F).
- les (méth)acrylamides de formule : $CH_2$=C(R')-CO-$NR_7R_8$,

où $R_7$ et $R_8$ identiques ou différents représentent un, atome d'hydrogène ou un groupe alkyle de 1 à 12 atomes de carbone linéaire ou ramifié, tel qu'un groupe n-butyle, t-butyle, isopropyle, isohexyle, isooctyle, ou isononyle, et R' désigne H ou méthyle.

**16.** Composition selon l'une des revendications précédentes, dans laquelle le copolymère à gradient comprend au

moins un monomère dont l'homopolymère a une Tg supérieure ou égale à 20°C, choisi parmi :

- les acrylates de furfuryle, d'isobornyle, de tertiobutyle, de tertiobutylecyclohexyle, de tertiobutylbenzyle;
- les méthacrylates de méthyle, de n-butyle, d'éthyle, d'isobutyle,
- le styrène, le styrène sulfonate;
- l'acétate de vinyle et le vinylcyclohexane.

**17.** Composition selon l'une des revendications précédentes, dans laquelle le copolymère à gradient est présent en une quantité de 0,1 à 60% en poids, de préférence 0,2 à 40% en poids, notamment 1 à 35% en poids, voire 5-30% en poids, par rapport au poids total de la composition.

**18.** Composition selon l'une des revendications précédentes, dans laquelle le copolymère à gradient est présent sous forme solubilisée, par exemple dans l'eau ou un solvant organique, ou bien sous forme de dispersion aqueuse ou organique.

**19.** Composition selon l'une des revendications précédentes, comprenant au moins un constituant choisi parmi les polymères filmogènes additionnels; les agent auxiliaires de filmification; l'eau; les solvants organiques; les épaississants; les matières colorantes; les charges, les agents d'étalement, les agents mouillants, les agents dispersants, les anti-mousses, les conservateurs, les filtres UV, les actifs, les tensioactifs, les agents hydratants, les parfums, les stabilisants, les antioxydants, les vitamines, les oligo-éléments, les agents alcalinisants ou acidifiants, les céramides, ou leurs mélanges.

**20.** Composition selon l'une des revendications précédentes, étant apte à former un film ayant une perte de brillance, après 10 secondes d'abrasion avec l'abrasimètre TABER, la brillance étant mesurée 1 heure après l'abrasion, inférieure ou égale à 14%, de préférence inférieure ou égale à 12%, préférentiellement inférieure ou égale à 8%.

**21.** Composition selon l'une des revendications précédentes, étant apte à former un film ayant un module d'Young allant de 10 à 200 MPa, de préférence de 10 à 100 MPa, et mieux de 10 à 50 MPa.

**22.** Composition selon l'une des revendications précédentes, se présentant sous la forme d'une base pour vernis, d'un produit de maquillage des ongles, d'une composition de finition ou "top-coat" à appliquer sur le produit de maquillage des ongles, d'un produit de soin cosmétique des ongles.

**23.** Procédé cosmétique de maquillage ou de soin des ongles comprenant l'application sur ceux-ci d'une composition cosmétique telle que définie selon l'une des revendications précédentes.

Office européen des brevets

# RAPPORT DE RECHERCHE EUROPEENNE

Numéro de la demande
EP 03 29 2652

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| D,A | MATYJASZEWSKI K ET AL: "Atom transfer radical polymerization" CHEMICAL REVIEWS, AMERICAN CHEMICAL SOCIETY. EASTON, US, vol. 101, no. 9, septembre 2001 (2001-09), pages 2921-2990, XP002212148 ISSN: 0009-2665 * page 2958 - page 2959 * --- | 1 | A61K7/043 |
| A | KRYSZEWSKI M: "GRADIENT POLYMERS AND COPOLYMERS" POLYMERS FOR ADVANCED TECHNOLOGIES, JOHN WILEY AND SONS, CHICHESTER, GB, vol. 9, no. 4, 1 avril 1998 (1998-04-01), pages 244-259, XP000766646 ISSN: 1042-7147 * page 258 * --- | 1 | |
| A | US 6 312 672 B1 (SHIRAZI FAHIMEH P ET AL) 6 novembre 2001 (2001-11-06) * colonne 1, ligne 8 - ligne 14 * * colonne 8, ligne 39 - ligne 43 * ----- | 1 | DOMAINES TECHNIQUES RECHERCHES (Int.Cl.7) A61K |

Le présent rapport a été établi pour toutes les revendications

| Lieu de la recherche | Date d'achèvement de la recherche | Examinateur |
|---|---|---|
| MUNICH | 2 mars 2004 | Mellgren, M |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
& : membre de la même famille, document correspondant

EPO FORM 1503 03.82 (P04C02)

## ANNEXE AU RAPPORT DE RECHERCHE EUROPEENNE RELATIF A LA DEMANDE DE BREVET EUROPEEN NO.

EP 03 29 2652

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

02-03-2004

| Document brevet cité au rapport de recherche | | Date de publication | Membre(s) de la famille de brevet(s) | | Date de publication |
|---|---|---|---|---|---|
| US 6312672 | B1 | 06-11-2001 | AU | 1442901 A | 14-05-2001 |
| | | | WO | 0132136 A1 | 10-05-2001 |

EPO FORM P0460

Pour tout renseignement concernant cette annexe : voir Journal Officiel de l'Office européen des brevets, No.12/82